# EUROPEAN PATENT SPECIFICATION

(11) **EP 1 784 147 B1**
(45) Date of publication and mention of the grant of the patent: **06.07.2016**
(21) Application number: 05790406.2
(22) Date of filing: 17.08.2005
(51) Int. Cl.: A61F 2/44, A61B 17/70, A61F 2/30, A61F 2/46, A61B 17/00

(54) **IMPLANTABLE SPINAL DEVICE REVISION SYSTEM**
IMPLANTIERBARES WIRBELSÄULEN-VORRICHTUNGS-REVISIONSSYSTEM
SYSTÈME DE RÉVISION DE DISPOSITIF SPINAL IMPLANTABLE

(30) Priority: 18.08.2004 US 602827 P; 13.01.2005 US 643556 P; 02.03.2005 US 71541
(43) Date of publication of application: 16.05.2007
(73) Proprietor: Gmedelaware 2 LLC, Wilmington DE 19801 (US)
(72) Inventor: BROMAN, Richard, Monroe, WA 98722 (US); MCLEER, Thomas, Redmond, WA 98053 (US); TOKISH, Leonard, Jr., Issaquah, WA 98929 (US); REILEY, Mark, A., Piedmont, CA 94611 (US); SUH, Sean, S., Redmond, WA 98052 (US)
(74) Representative: Potter Clarkson LLP
(86) International application number: PCT/US2005/029476
(87) International publication number: WO 2006/023683

(56) References cited:
- EP-A- 1 254 639
- WO-A-01/30248
- WO-A2-2006/055186
- FR-A- 2 844 180
- US-A- 5 662 651
- US-A- 5 683 391
- US-A- 5 885 286
- US-A1- 2003 181 914
- US-A1- 2005 240 265

## Description

### FIELD OF THE INVENTION

The present invention generally relates to devices for treatment of various spinal pathologies. More specifically, the present invention is directed to configurable and anatomically adaptable implantable devices for use in a spine and surgical procedures for altering the biomechanics of a spine, either temporarily or permanently. The devices alter, replace and/or revise existing anatomy and/or previously implanted devices.

### BACKGROUND OF THE INVENTION

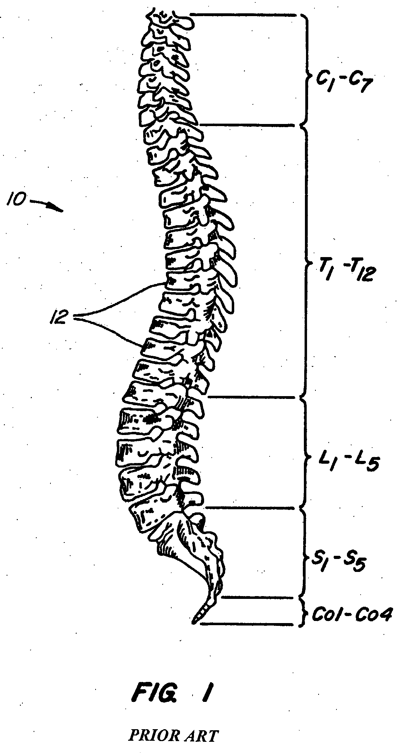

Back pain, particularly in the small of the back, or lumbosacral region (L4-S 1) of the spine (*see,* **FIG. 1**), is a common ailment. In many cases, the pain severely limits a person's functional ability and quality of life. Back pain interferes with work, routine daily activities, and recreation. It is estimated that Americans spend $50 billion each year on low back pain alone. It is the most common cause of job-related disability and a leading contributor to missed work.

Through disease or injury, the laminae, spinous process, articular processes, facets and/or facet capsules of one or more vertebral bodies along with one or more intervertebral discs can become damaged which can result in a loss of proper alignment or loss of proper articulation of the vertebra. This damage can result in an anatomical change, loss of mobility, and pain or discomfort. For example, the vertebral facet joints can be damaged by traumatic injury or as a result of disease. Diseases damaging the spine and/or facets include osteoarthritis where the cartilage of joints is gradually worn away and the adjacent bone is remodeled, ankylosing spondylolysis (or rheumatoid arthritis) of the spine which can lead to spinal rigidity, and degenerative spondylolisthesis which results in a forward displacement of the lumbar vertebra on the sacrum. Damage to facet joints of the vertebral body often results in pressure on nerves, commonly referred to as "pinched" nerves, or nerve compression or impingement. The result is pain, misaligned anatomy, a change in biomechanics and a corresponding loss of mobility. Pressure on nerves can also occur without facet joint pathology, *e.g*., as a result of a herniated disc.

One conventional treatment of facet joint pathology is spine stabilization, also known as intervertebral stabilization. Intervertebral stabilization desirably controls, prevents or limits relative motion between the vertebrae, through the use of spinal hardware, removal of some or all of the intervertebral disc, fixation of the facet joints, bone graft/osteo-inductive/osteo-conductive material positioned between the vertebral bodies (with or without concurrent insertion of fusion cages), and/or some combination thereof, resulting in the fixation of (or limiting the motion of) any number of adjacent vertebrae to stabilize and prevent/limit/control relative movement between those treated vertebrae.

Although spine fusion surgery is an efficacious treatment alternative, complications can, nonetheless, result. Patients undergoing spine surgery frequently continue to experience symptoms. For surgical procedures in the lumbar spine, failure rates as high as 37% have been reported after lumbar fusion and 30% for surgery without fusion. *See* Eichholz, et al., "Complications of Revision Spinal Surgery," Neurosurg Focus 15(3):1-4 (2003). Post-operative problems can include: decompression related problems, and fusion related problems. Decompression related problems (*i.e.*, loss of normal spine balance resulting in the head and trunk no longer being centered over the pelvis) include, for example, recurrent disc herniation, spinal stenosis, chronic nerve injury, infection, and decompression. Fusion related problems can include, pain from the bone harvest site, failure of a fusion to develop, loosening of the implanted devices, nerve irritation caused by the devices, infection, and poor alignment of the spine.

Stabilization of vertebral bodies can also be achieved (to varying degrees) from a wide variety of procedures, including the insertion of motion limiting devices (such as intervertebral spacers, artificial ligaments and/or dynamic stabilization devices), devices promoting arthrodesis (rod and screw systems, cables, fusion cages, etc.), and complete removal of some or all of a vertebral body from the spinal column (which may be due to extensive bone damage and/or tumorous growth inside the bone) and insertion of a vertebral body replacement (generally anchored into the adjacent upper and lower vertebral bodies). Various devices are known for fixing the spine and/or sacral bone adjacent the vertebra, as well as attaching devices used for fixation, including devices disclosed in: U.S. Patent Nos. 6,585,769; 6,290,703; 5,782,833; 5,738,585; 6,547,790; 6,638,321; 6,520,963; 6,074,391; 5,569,247; 5,891,145; 6,090,111; 6,451,021; 5,683,392; 5,863,293; 5,964,760; 6,010,503; 6,019,759; 6,540,749; 6,077,262; 6,248,105; 6,524,315; 5,797,911; 5,879,350; 5,885,285; 5,643,263; 6,565,565; 5,725,527; 6,471,705; 6,554,843; 5,575,792; 5,688,274; 5,690,630; 6,022,350; 4,805,602; 5,474,555; 4,611,581; 5,129,900; 5,741,255; 6,132,430; and U.S. Patent Publication No. 2002/0120272.

More recently, various treatments have been proposed and developed as alternatives to spinal fusion Many of these treatments seek to restore (and/or maintain) some, or all, of the natural motion of the treated spinal unit, and can include intervertebral disc replacement, nucleus replacement, facet joint resurfacing, and facet joint replacement. Such solutions typically include devices that do not substantially impair spinal movement. See, U.S. Patent Nos. 6,610,091; 6,811,567; 6,902,580; 5,571,171; and Re 36,758; and PCT Publication Nos. WO 01/158563, WO 2004/103228, WO 2005/009301, and WO 2004/103227. Thus, spinal arthroplasty has become an acceptable alternative to fusion, particularly in cases of degenerative disc disease. Arthroplasty devices can be particularly useful because the devices are designed to create an artificial joint or restore the functional integrity and power of a joint.

One device developed to treat patients with, for example, lumbar degenerative disc disease, is the Charité III artificial disc (DePuy Spine, a Johnson & Johnson Company), a device that replaces the natural intervertebral disc ***34*.** Devices, such as the Charité are comprised of suitable orthopedic and biocompatible materials such as cobalt chromium and ultra-high molecular weight polyethylene (UHMWPE). The devices are designed to enable independent translation and rotation, which is a component of physiological motion. See, for example, U.S. Patent Nos. 6,793,678 and 6,770,095. In other instances, where a disc is removed, *e.g.* to treat a prolapsed disc, a wedge can be placed within the empty disc space to compensate for the removed natural disc and to support the adjoining vertebral bodies. Further, a plate and screws may be used to hold the wedge in place, such as with an anterior cervical decompression and fusion system.

WO 01/30248 describes devices and surgical methods for treating spinal pathologies including degenerative spondylolisthesis, spinal stenosis, degenerative lumbar scoliosis and kypho-scoliosis. A universal facet prosthesis includes a cup member fixed to a stem and a separate surface member that is held within the cup member.

### SUMMARY OF THE INVENTION

According to the invention, there is provided a spinal facet joint prothesis as defined in the appended Claim 1.

Preferred embodiments are defined in the dependent claims.
Once the initial surgical treatment and implantation has been completed for any of these spinal devices (and their related surgical techniques), additional problems and/or complications, such as additional disc problems, future disc degeneration, stenosis, pseudoarthrosis, junctional failure of the spine, failure of the implant and/or additional nerve compression can occur. In some cases, problems can occur much later, even years later. These late onset complications can include, for example, further need for decompression, the onset of other spinal degeneration, requirements for revision of the spinal construct and/or need for fusion of the affected spinal motion segment(s).

Regardless of whether the complications result from decompression or from complications arising after the surgery, revision surgery is sometimes required. Further, in some instances, it may be desirable to convert a spinal pathology that has been treated with, for example, an arthroplasty device that restores motion to the joint to a fusion device that limits motion within the joint. This can particularly be true for patients that have required surgical intervention at an early age.

Part of the invention disclosed herein includes the realization that there exists a need for devices that facilitate revision spinal surgery, desirably with minimal disruption to areas that have already undergone spinal surgery. Needed devices include devices that alter the biomechanics of a joint, either temporarily or permanently, devices that replace and/or repair all or selected portions of an existing device, and devices that address complications or further spinal degeneration that have arisen since the initial surgical intervention.

Disclosed herein is an implantable arthroplasty device revision system, components of which are configured for implantation in conjunction with an arthroplasty device and a first vertebra and a second vertebra comprising: a spine reconstruction device for replacing bone comprising an elongated tubular member with an anchoring member on a portion of an exterior of the elongated tubular member, and an aperture adapted to communicate with a bone surface; a revision cap adapted to mate with a truncated stem of an implanted arthroplasty device comprising a cap adapted to mate with a stem of the implanted arthroplasty device and an arthroplasty device receiving housing connected to the cap; a revision stem comprising a stem having a cap at an end of the stem and an arthroplasty device receiving housing connected to the cap; a modular cephalad stem having an auxiliary sleeve adapted to receive a threaded female stem, a male stem, and a connector; a cross-linking arm having a length adapted to fit between a pair of cephalad stems, each end of which is adapted to connect to a cephalad arm; and an arthroplasty device joint controller adapted to control movement of an arthroplasty device joint having a base adapted to engage a device joint at a first location, a side and a top adapted to engage the device joint at a second location.

Also disclosed herein are methods for revising an implanted device for treating a spinal pathology. The methods provide for altering the existing biomechanics of the spine, either permanently or temporarily.

In various embodiments of the invention, a facet joint replacement device is provided for implantation on a vertebral body to replace a portion of the natural facet joint. The implanted device is revised using a securing device of the invention installed on a joint of the facet joint replacement device. The securing cap, or locking cap, prevents and/or limits articulation of the ball and cup joint of the arthroplasty device, converting the device to the equivalent of a spinal fusion device.

The implanted articulating joint device is revised by removing portions of the device and replacing the removed portions or components with components of the invention that secure or lock the remaining elements together, achieving an equivalent, or substantial equivalent, of a fusion device's functionality.

The replacement components or devices are adapted to the pre-existing implanted arthroplasty devices such that some or all of the existing bone anchors do not need to be removed and/or disturbed to convert the articulating arthroplasty device to a device with controlled, limited and/or no movement.

The present disclosure further includes an implantable device for revising an implanted spinal arthroplasty device comprising a first surface adapted to communicate with an anatomical surface of the spine; and a second surface adapted to engage a portion of the implanted spinal arthroplasty device. In some embodiments, the first surface is configured to communicate with a revised anatomical surface. In other devices, the revision device has threads adapted to engage the anatomical surface at a first end. The threads can be positioned on an exterior surface of the revision device. Additionally, a hollow aperture for receiving a connector of the arthroplasty device can be provided. Where a hollow aperture is provided the aperture can be configured such that it is internally threaded to receive a connector of the arthroplasty device. The revision device can also be adapted to deliver bone cement to the anatomical surface. Additionally, or in the alternative, the revision device can be adapted at an end to engage an arthroplasty device. The devices can be adapted to alter the biomechanics of the arthroplasty device, either permanently, semi-permanently, or temporarily. The revision device can be configured to secure the arthroplasty device and/or prevent movement of the arthroplasty device with respect to the anatomical surface(s) to which it is connected.

The present disclosure further includes an implantable device for altering the biomechanics of an implanted spinal arthroplasty device comprising: a first surface adapted to communicate with an anatomical surface; and a second surface adapted to engage a portion of the arthroplasty device. The revision device can also be configured to provide threads to engage the natural anatomical surface at a first end. Threads can be positioned on an interior and/or exterior of the device. The hollow aperture can be configured to receive a connector of the arthroplasty device. The revision device is adapted to deliver bone cement to the anatomical surface. Additionally, the device can be configured to engage an arthroplasty device and/or alter the biomechanics of the arthroplasty device. Where the biomechanics are altered, such alteration can be made permanently, semi-permanently, or temporarily. The revision device can also be configured to secure the arthroplasty device, to prevent movement of the device with respect to the natural anatomical surface.

In yet another disclosed device, the device includes an implantable spinal arthroplasty device revision system, components of which are configured for implantation in conjunction with a spinal arthroplasty device and a first and second vertebra of a spine, comprising at least one of: a spine reconstruction device for replacing bone comprising an elongated tubular member with an anchoring member on a portion of an exterior of the elongated tubular member, an aperture adapted to communicate with a bone surface, and a proximal end adapted to replace a mating surface; a revision cap adapted to mate with a truncated stem of an implanted arthroplasty device comprising a cap adapted to mate with a stem of the implanted arthroplasty device and an arthroplasty device receiving housing connected to the cap; a revision stem comprising a stem adapted to be implanted within bone and having a cap at an end of the stem and an arthroplasty device receiving housing connected to the cap; a modular cephalad stem having an auxiliary sleeve adapted to receive a threaded female stem, a male stem, and a connector; a cross-linking arm having a length adapted to fit between a pair of cephalad arms of an arthroplasty device, each end of which is adapted to connect to a cephalad arm; and an arthroplasty device joint controller adapted to control movement of an arthroplasty device joint having a base adapted to engage a device joint at a first location, a side and a top adapted to engage the device joint at a second location. The device can also include an artificial disc, intervertebral wedges, bone filler, bone cement, and biocompatible adhesive. Additionally, in some devices, the restoration units can be internally and/or externally threaded. The restoration unit(s) can be configured such that it is adapted to replace a spine anatomy, such as pedicle, lamina, spinous processes, other processes and/or the vertebral body. The restoration units are adapted to connect to an arthroplasty device. Thus, in at least some devices, the arthroplasty device receiving housing is positioned adjacent the cap and/or the housing is adapted to connect to an element of an implanted arthroplasty device. In some devices, the revision cap is a polyaxial element Further embodiments can include a configuration wherein the housing moves relative to the cap by a ball and socket connector. The housing can be rotatably connected to the revision cap. The revision cap can be adapted to engage the implanted arthroplasty device. In some devices, it may be desirable to provide for internal and/or external threading of the sleeve. A female aperture forming a keyway can also be provided. Additionally, the male stem can be configured to have a male protrusion adapted to fit within a configured female aperture of the auxiliary sleeve. The modular cephalad stem can be adapted in some devices to provide anti-rotation of the male stem to the female auxiliary sleeve. Further, the modular stem can include a securing member. The base of the joint controller can be positioned on the arthroplasty device joint opposite a position of the top of the joint controller. The revision system can also be configured so that the joint controller snap fits over the joint of the arthroplasty device. In other devices, the joint controller has an aperture on the top of the device, which can further be adapted to receive a securing mechanism.

In yet another disclosed device, an implantable device for restoring a target surface area of a vertebral body, the implantable device comprising an elongated tubular member with an anchoring member on a portion of an exterior of the elongated tubular member at a first end, and an aperture adapted to communicate with a tissue and an aperture adapted to communicate with an implantable arthroplasty device at a second end.

In yet another disclosed device, an implantable device is provided for revising a previously implanted arthroplasty device having a fixation element, the implantable device comprising a cap adapted to mate with a stem of the previously implanted fixation element, and a housing connected to the cap on a first end and adapted to engage an element of an arthroplasty device on a second end.

Another disclosed device includes an implantable device for use with an arthroplasty device, the implantable device comprising a stem having a tapered first end, and a housing adapted to engage an element of the arthroplasty device at a second end.

Yet another disclosed device provides an implantable device for use with an arthroplasty device, the implantable device comprising a modular stem having a first stem component with a male end, and a second stem component with a female end, wherein the male end is adapted to fit within the female end to prevent rotation and/or relative movement.

In still another an implantable device for use with an arthroplasty device, the implantable device comprising a cross-linking arm adapted to connect to a first arm of the arthroplasty device at a first end and a second arm of the joint arthroplasty device at a second end is provided.

In yet another disclosed device, an implantable device for use with an arthroplasty device comprising a lock adapted to engage a joint of the arthroplasty device to reduce, control, modify and/or prevent articulation of the joint.

Also disclosed herein is a method of revising an implanted arthroplasty device, the method comprising: accessing an implanted spinal arthroplasty device; and inserting a revision device adapted to alter the biomechanics of the implanted spinal arthroplasty device. Where methods are employed, the revision device can be configured to restore the operation of the implanted arthroplasty device. Alternatively, or additionally, the revision device can be configured to limit and/or modify the operation of the implanted arthroplasty device. In practicing the method of the invention, the implanted arthroplasty device can be converted to a fusion device. Revision can be achieved at the time of implantation of the arthroplasty device or at a subsequent time. When performing the methods disclosed herein, a user will select, as many times as desirable, from a plurality of devices suitable for revising the arthroplasty device.

### BRIEF DESCRIPTION OF THE DRAWINGS

The features of the invention are set forth with particularity in the appended claims. A better understanding of the features and advantages of the present invention will be obtained by reference to the following detailed description that sets forth illustrative devices, in which the principles of the invention are utilized, and the accompanying drawings of which:
**FIG. 1** is a lateral elevation view of a normal human spinal column;
**FIG. 2** is a superior view of a normal human lumbar vertebra;
**FIG. 3** is a lateral elevational view of two vertebral bodies forming a functional spinal unit;
**FIG. 4** is a posterolateral oblique view of a vertebrae from a human spinal column;
**FIG. 5** is a perspective view of the anatomical planes of the human body;
**FIG. 6** is a perspective view of an implantable configurable modular spinal arthroplasty device;
**FIG. 7** is a perspective view of the implantable configurable modular spinal arthroplasty device shown in **FIG. 6****,** implanted;
**FIG. 8** is a posterior view of an implantable configurable spinal arthroplasty device for replacing resected facet joints;
**FIG. 9** is a perspective view of a restoration device for replacing or augmenting a target bone structure;
**FIG. 10A** illustrates the device illustrated in **FIG. 9** implanted within a vertebral body; **FIG. 10B** illustrates a vertebral body with two devices implanted therein;
**FIG. 11A** illustrates a restoration device implanted within in a vertebral body in combination with an spinal arthroplasty device; **FIG. 11B** illustrates a restoration device implanted with a facet repair device; **FIG. 11C** illustrates a restoration device implanted with a device for replacing resected facet joints; **FIG.11D** illustrates a restoration device implanted with an arthroplasty device providing a surface for the cephalad arm(s);
**FIG. 12A** illustrates an alternate design of a revision device featuring an adapter cap for receiving a portion of an implanted spinal arthroplasty device; **FIG.12B** is a device having an offset adapter cap; **FIG.12C** illustrates a device implanted into a vertebral body such that the cap of the device can function as a pedicle replacement;
**FIG.13A** illustrates an alternate configuration of a device of **FIG.12** having an alternate design for the adapter cap; **FIG.13B** illustrates the device shown in **FIG.13A** implanted in a vertebral body;
**FIG.14A** illustrates an adapter cap implanted in combination with an arthroplasty device; **FIG.14B** illustrates an adapter cap implanted in combination with another arthroplasty device; **FIG.14C** illustrates an adapter cap implanted in combination with the facet replacement device of **FIG.8**;
**FIG.15A** illustrates a perspective view of component parts of a replaceable modular stem system for use in an implantable spinal arthroplasty device; **FIG.15B** illustrates a side view of replaceable modular stem system;
**FIG 16A** illustrates side view of a replaceable modular stem system of an alternate device; **FIG.16B** illustrates a cross-section of the modular system depicted in **FIG. 16A; FIG. 16C** illustrates an optional tie-down connector in combination with the system;
**FIG. 17** illustrates an alternate modular stem system having a bushing;
**FIG. 18** illustrates the modular stem system of **FIG. 17** connected to a housing;
**FIG. 19** illustrates an implanted arthroplasty device having a cross-linking arm installed;
**FIG. 20A** illustrates a securing device for use in connection with an arthroplasty device to revise and/or modify, control, or limit motion of the arthroplasty device; **FIG. 20B** is a top view of the securing device; **FIG. 20C** is a side view of the securing device; **FIG. 20D** is a bottom view of the securing device; **FIG. 20E** is a cross-sectional view of the securing device;

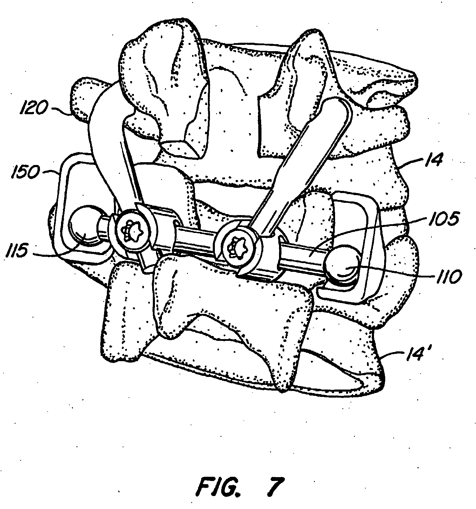

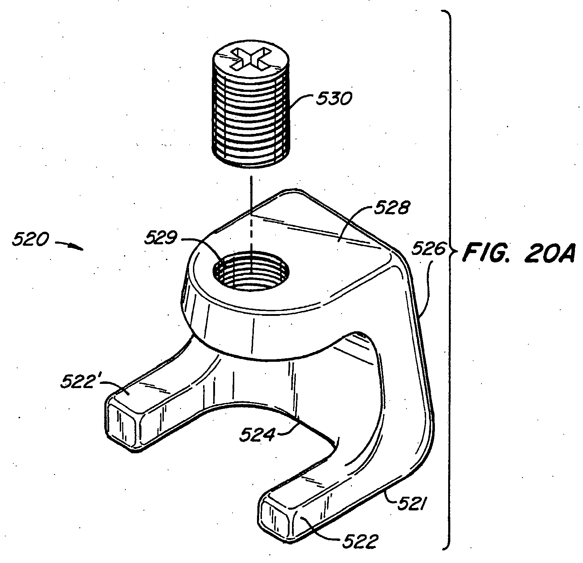

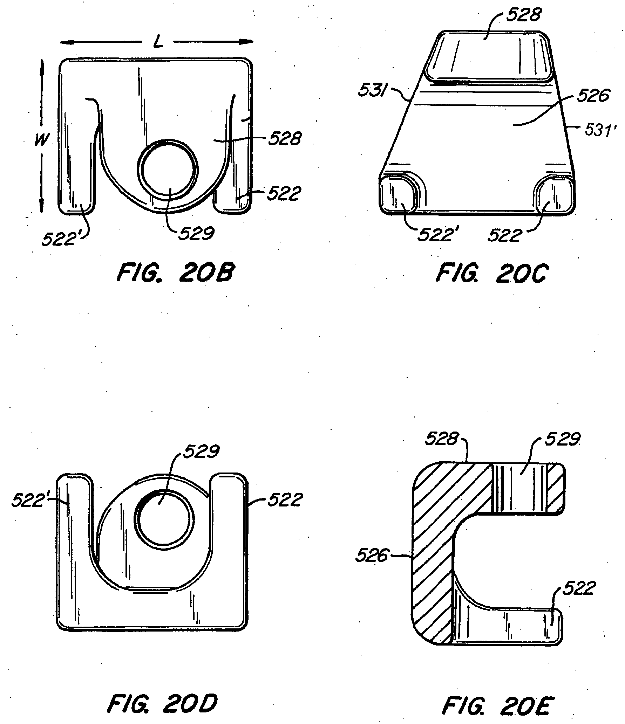

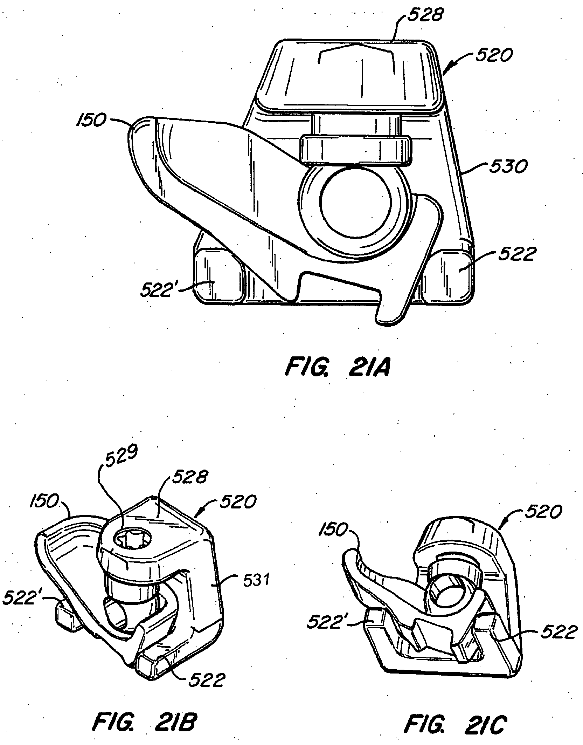

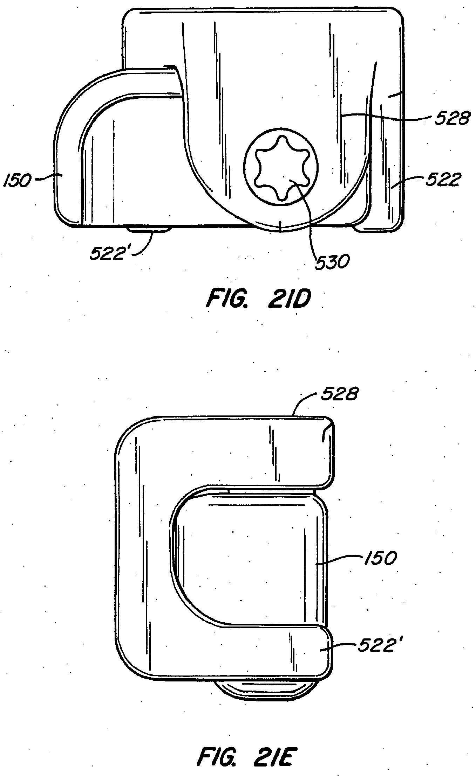

**FIG. 21A** illustrates a side view of the securing device of **FIG. 20** in combination with a portion of the arthroplasty device of **FIG. 7****;** **FIG. 21B** illustrates a perspective view of the securing device in combination with a portion of the arthroplasty device; **FIG. 21C** is a perspective view from an anterior perspective of the securing device in combination with a portion of the arthroplasty device; **FIG. 21D** is a top view of the securing device with a portion of the arthroplasty device; **FIG. 21E** is a bottom view of the securing device with a portion of the arthroplasty device;
**FIG. 22A** is a perspective view of an implanted arthroplasty device with the securing device of **FIG. 20****;** **FIG. 22B** is a perspective view of another implanted arthroplasty device with the securing device of **FIG. 20****;** **FIG. 22C** is a perspective view of yet another implanted arthroplasty device with the securing device of **FIG. 20****;**
**FIGS. 23** illustrates a caudal cup incorporating a flange and a compression device to control movement of the cross-member of an arthroplasty device;
**FIG. 24A-D** illustrates modifications to the caudal cup of an arthroplasty device to prevent dislocation and/or alter the biomechanics of the arthroplasty device; and
**FIG. 25** is a flow chart illustrating the methods of revising the biomechanics of a patient having an implantable device.

### DETAILED DESCRIPTION OF THE INVENTION

The invention relates generally to implantable devices, apparatus or mechanisms that are suitable for implantation within a human body to restore, augment, and/or replace soft tissue and connective tissue, including bone and cartilage, and systems for treating spinal pathologies. In some instances the implantable devices can include devices designed to replace missing, removed or resected body parts or structure. The implantable devices, apparatus or mechanisms are configured such that the devices can be formed from parts, elements or components which alone or in combination comprise the device. The implantable devices can also be configured such that one or more elements or components are formed integrally to achieve a desired physiological, operational or functional result such that the components complete the device. Functional results can include the surgical restoration and functional power of a joint, controlling, limiting or altering the functional power of a joint, and/or eliminating the functional power of a joint by preventing joint motion. Portions of the device can be configured to replace or augment existing anatomy and/or implanted devices, and/or be used in combination with resection or removal of existing anatomical structure.

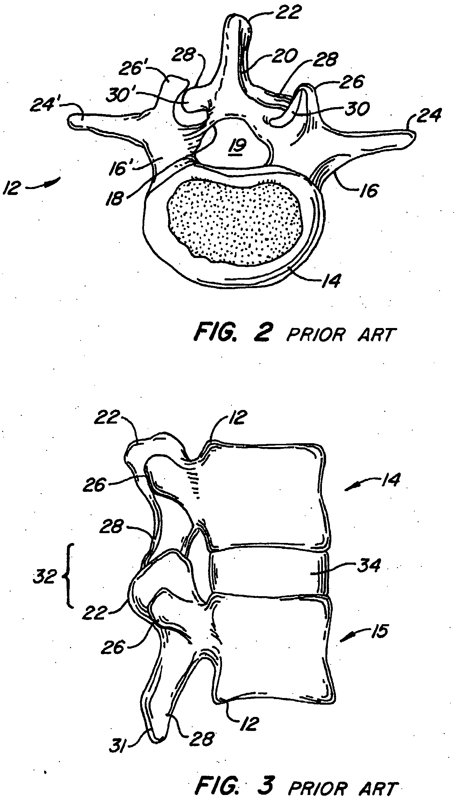

The devices of the invention are designed to interact with the human spinal column ***10**,* as shown in **FIG. *1******,*** which is comprised of a series of thirty-three stacked vertebrae ***12*** divided into five regions. The cervical region includes seven vertebrae, known as **C1-C7.** The thoracic region includes twelve vertebrae, known as **T1-T12.** The lumbar region contains five vertebrae, known as **L1-L5.** The sacral region is comprised of five fused vertebrae, known as **S1-S5,** while the coccygeal region contains four fused vertebrae, known as **Co1-Co4.** An example of one of the vertebra is illustrated in **FIG. 2** which depicts a superior plan view of a normal human lumbar vertebra ***12.*** Although human lumbar vertebrae vary somewhat according to location, the vertebrae share many common features. Each vertebra ***12*** includes a vertebral body ***14.*** Two short boney protrusions, the pedicles ***16, 16',*** extend dorsally from each side of the vertebral body ***14*** to form a vertebral arch ***18*** which defines the vertebral foramen ***19.*** At the posterior end of each pedicle ***16,*** the vertebral arch ***18*** flares out into broad plates of bone known as the laminae ***20.*** The laminae ***20*** fuse with each other to form a spinous process ***22.*** The spinous process ***22*** provides for muscle and ligamentous attachment. A smooth transition from the pedicles ***16*** to the laminae ***20*** is interrupted by the formation of a series of processes.

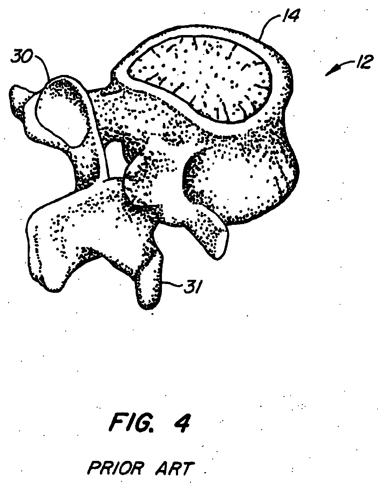

Two transverse processes ***24, 24'*** thrust out laterally, one on each side, from the junction of the pedicle ***16*** with the lamina ***20.*** The transverse processes ***24, 24'*** serve as levers for the attachment of muscles to the vertebrae ***12.*** Four articular processes, two superior ***26*, *26'*** and two inferior **28, 28',** also rise from the junctions of the pedicles ***16*** and the laminae ***20.*** The superior articular processes ***26, 26'*** are sharp oval plates of bone rising upward on each side of the vertebrae, while the inferior processes ***28, 28'*** are oval plates of bone that jut downward on each side. *See also* **FIG. 4**.

The superior and inferior articular processes ***26*** and ***28*** each have a natural bony structure known as a facet. The superior articular facet ***30*** faces medially upward, while the inferior articular facet ***31*** (see **FIG. 3**) faces laterally downward. When adjacent vertebrae ***12*** are aligned, the facets ***30*, *31,*** which are capped with a smooth articular cartilage and encapsulated by ligaments, interlock to form a facet joint 32. The facet joints are apophyseal joints that have a loose capsule and a synovial lining.

As discussed, the facet joint ***32*** is comprised of a superior facet and an inferior facet (shown in **FIG. 4**). The superior facet is formed in the vertebral level below the joint ***32,*** and the inferior facet is formed in the vertebral level above the joint ***32.*** For example, in the **L4-L5** facet joint shown in **FIG**. **3**, the superior facet of the joint ***32*** is formed by bony structure on the ***L5*** vertebra *(i.e.,* a superior articular surface and supporting bone ***26*** on the **L5** vertebra), and the inferior facet of the joint ***32*** is formed by bony structure on the **L4** vertebra (*i.e.,* an inferior articular surface and supporting bone ***28*** on the **L4** vertebra). The angle formed by a facet joint located between a superior facet and an inferior facet changes with respect to the midline depending upon the location of the vertebral body along the spine. The facet joints do not, in and of themselves, substantially support axial loads unless the spine is in an extension posture (lordosis). As would be appreciated by those of skill in the art, the orientation of the facet joint for a particular pair of vertebral bodies changes significantly from the thoracic to the lumbar spine to accommodate a joint's ability to resist flexion-extension, lateral bending, and rotation.

An intervertebral disc ***34*** located between each adjacent vertebra ***12*** (with stacked vertebral bodies shown as ***14*, *15*** in **FIG. 3**) permits gliding movement between the vertebrae ***12.*** The structure and alignment of the vertebrae ***12*** thus permit a range of movement of the vertebrae ***12*** relative to each other. **FIG. 4** illustrates a posterolateral oblique view of a vertebrae ***12,*** further illustrating the curved surface of the superior articular facet ***30*** and the protruding structure of the inferior facet ***31*** adapted to mate with the opposing superior articular facet. As discussed above, the position of the inferior facet ***31*** and superior facet ***30*** varies on a particular vertebral body to achieve the desired biomechanical behavior of a region of the spine.

Thus, overall the spine comprises a series of functional spinal units that are a motion segment consisting of two adjacent vertebral bodies, the intervertebral disc, associated ligaments, and facet joints. *See* Posner, I, et al. "A biomechanical analysis of the clinical stability of the lumbar and lumbosacral spine." Spine 7:374-389 (1982).

As previously described, a natural facet joint, such as facet joint ***32* (****FIG. 3**), has a superior facet ***30*** and an inferior facet ***31.*** In anatomical terms, the superior facet of the joint is formed by the vertebral level below the joint, which can thus be called the "caudal" portion of the facet joint because it is anatomically closer to the tail bone or feet of the person. The inferior facet of the facet joint is formed by the vertebral level above the joint, which can be called the "cephalad" portion of the facet joint because it is anatomically closer to the head of the person. Thus, a device that, in use, replaces the caudal portion of a natural facet joint (*i.e.,* the superior facet ***30***) can be referred to as a "caudal" device. Likewise, a device that, in use, replaces the cephalad portion of a natural facet joint *(i.e.,* the inferior facet ***31***) can be referred to a "cephalad" device.

When the processes on one side of a vertebral body ***14*** are spaced differently from processes on the other side of the same vertebral body, components of the devices on each side would desirably be of differing sizes as well to account for anatomical difference that can occur between patients. Moreover, it can be difficult for a surgeon to determine the precise size and/or shape necessary for an implantable device until the surgical site has actually been prepared for receiving the device. In such case, the surgeon typically can quickly deploy a family of devices possessing differing sizes and/or shapes during the surgery. Thus, embodiments of the spinal devices of the present invention include modular designs that are either or both configurable and adaptable. Additionally, the various embodiments disclosed herein may also be formed into a "kit" or system of modular components that can be assembled *in situ* to create a patient specific solution. As will be appreciated by those of skill in the art, as imaging technology improves, and mechanisms for interpreting the images (*e.g*., software tools) improve, patient specific designs employing these concepts may be configured or manufactured prior to the surgery. Thus, it is within the scope of the invention to provide for patient specific devices with integrally formed components that are preconfigured.

A configurable modular device design, such as the one enabled by this invention, allows for individual components to be selected from a range of different sizes and utilized within a modular device. One example of size is to provide caudal and cephalad stems of various lengths. A modular implantable device design allows for individual components to be selected for different functional characteristics as well. One example of function is to provide stems having different surface features and/or textures to provide anti-rotation capability. Other examples of the configurability of modular implantable device of the present invention as described in greater detail below.

Implantable devices of the present invention are configurable such that the resulting implantable spinal device is selected and positioned to conform to a specific anatomy or desired surgical outcome. The adaptable aspects of embodiments of the present invention provide the surgeon with customization options during the implantation or revision procedure. It is the adaptability of the present device systems that also provides adjustment of the components during the implantation procedure to ensure optimal conformity to the desired anatomical orientation or surgical outcome. An adaptable modular device of the present invention allows for the adjustment of various component-to-component relationships. One example of a component-to-component relationship is the rotational angular relationship between a crossbar mount and the crossbar. Other examples of the adaptability of modular device of the present invention as described in greater detail below. Configurability may be thought of as the selection of a particular size of component that together with other component size selections results in a "custom fit" implantable device. Adaptability then can refer to the implantation and adjustment of the individual components within a range of positions in such a way as to fine tune the "custom fit" devices for an individual patient. The net result is that embodiments of the modular, configurable, adaptable spinal device and systems of the present invention allow the surgeon to alter the size, orientation, and relationship between the various components of the device to fit the particular needs of a patient during the actual surgical procedure.

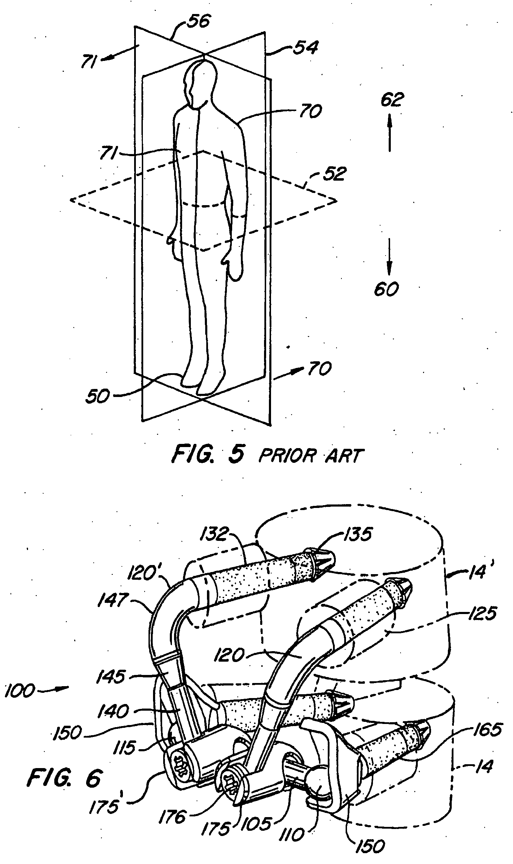

In order to understand the configurability, adaptability and operational aspects of the invention, it is helpful to understand the anatomical references of the body ***50*** with respect to which the position and operation of the devices, and components thereof, are described. There are three anatomical planes generally used in anatomy to describe the human body and structure within the human body: the axial plane ***52,*** the sagittal plane ***54*** and the coronal plane ***56*** (see **FIG. 5**). Additionally, devices and the operation of devices are better understood with respect to the caudal ***60*** direction and/or the cephalad direction ***62.*** Devices positioned within the body can be positioned dorsally ***70*** (or posteriorly) such that the placement or operation of the device is toward the back or rear of the body. Alternatively, devices can be positioned ventrally ***71*** (or anteriorly) such that the placement or operation of the device is toward the front of the body. Various embodiments of the spinal devices and systems of the present invention may be configurable and variable with respect to a single anatomical plane or with respect to two or more anatomical planes. For example, a component may be described as lying within and having adaptability in relation to a single plane. For example, a stem may be positioned in a desired location relative to an axial plane and may be moveable between a number of adaptable positions or within a range of positions. Similarly, the various components can incorporate differing sizes and/or shapes in order to accommodate differing patient sizes and/or anticipated loads.

Turning now to **FIG. 6**, an isometric view of a modular, configurable and adaptable implantable spinal arthroplasty device ***100*** is depicted. The spinal arthroplasty device ***100*** is illustrated implanted into vertebral bodies ***14.***

The arthroplasty device ***100*** and the various revision devices disclosed herein can be formed of a variety of materials. For example, where the devices have bearing surfaces (*i.e*. surfaces that contact another surface), the surfaces may be formed from biocompatible metals such as cobalt chromium steel, surgical steel, titanium, titanium alloys, tantalum, tantalum alloys, aluminum, *etc.* Suitable ceramics and other suitable biocompatible materials known in the art can also be used. Suitable polymers include polyesters, aromatic esters such as polyalkylene terephthalates, polyamides, polyalkenes, poly(vinyl) fluoride, PTFE, polyarylethyl ketone, and other materials that would be known to those of skill in the art. Various spinal arthroplasty device could comprise a flexible polymer section (such as a biocompatible polymer) that is rigidly or semi rigidly fixed to the adjacent vertebral bodies whereby the polymer flexes or articulates to allow the vertebral bodies to articulate relative to one another.

The spinal arthroplasty device ***100*** includes a crossbar ***105,*** a pair of cephalad arms ***120, 120'*** and a pair of caudal arms ***150, 150'.*** In this exemplary device the facets of the spine (*see* **FIG. 4**, ***30***) are replaced by the cooperative operation of the crossbar ***105*,** the cephalad arms ***120, 120'*** and the adaptable crossbar mounts ***175, 175'*** that join the cephalad arms ***120, 120'*** to the crossbar 105, interacting with the caudal arms ***150, 150'*** which form cups to receive the crossbar ***105**.* The components of the spinal facet arthroplasty device ***100*** are designed to provide appropriate configurability and adaptability for the given disease state, patient specific anatomy and spinal level where the implant occurs.

The crossbar ***105*** has a first end ***110*** and a second end ***115.*** In the illustrated device, the crossbar ***105*** is a two piece bar where the first end ***110*** is attached to a threaded male portion having threads. The crossbar second end ***115*** is attached to a threaded female portion sized to receive the threads. The threaded ends allow for the width of the crossbar to be adjusted to mate with the width between caudal bearings ***150.*** Additional alternative configurations of the crossbar ***105*** could include a series of solid crossbars of varying widths and/or thicknesses, or an adjustable crossbar having some form of locking or biasing mechanism (such as a spring-loaded tensioner or detent mechanism, etc.).

A pair of cephalad arms ***120, 120'*** are also illustrated in the exemplary device configurable and adaptable spinal arthroplasty device ***100.*** Each cephalad arm ***120, 120'*** includes a bone engaging end ***125, 125'*** and an end ***140*** adapted to couple to the crossbar ***105**.* The cephalad end ***140*** is adapted to engage the crossbar ***105*** and includes an arm ***145*** and an elbow ***147.*** The cephalad end ***140*** is attached to the crossbar using the crossbar mount ***175.*** The bone engaging end ***125*** includes a cephalad stem ***130*** and a distal tip ***135**.* The cephalad stem ***130*** and the distal tip ***135*** are threaded or otherwise configured to engage. Alternatively, the distal tip ***135*** could be formed integrally with the cephalad stem ***130**,* of the same or a different material as the cephalad stem ***130.*** In the illustrated embodiment of the cephalad stem ***130**,* surface features ***132*** are provided. Surface features ***132*** can be, for example, a textured surface or other surface such as, for example, surface features to assist in bony in-growth. Similarly, the illustrated distal tip ***135*** can have surface features ***137.***

The crossbar mount ***175*** is a connection structure to couple the cephalad arms ***120, 120'*** to the crossbar ***105.*** In the illustrated device, the crossbar mount ***175*** includes a cephalad arm engaging portion ***172,*** a crossbar engaging portion ***174*** and a fixation element ***176.*** Fixation element can be a screw, stem, cork-screw, wire, staple, adhesive, bone, and other material suitably adapted for the design. As will be described in greater detail below, the crossbar mount ***175*** provides adaptability between the cephalad elements ***120*** and the crossbar ***105*** and the loading characteristics of the crossbar ends ***110, 115*** and the caudal cups ***150, 150'.*** **FIG. 7** illustrates a perspective view of the implantable arthroplasty device of **FIG. 6** implanted with respect to two vertebral bodies ***14, 14'.***

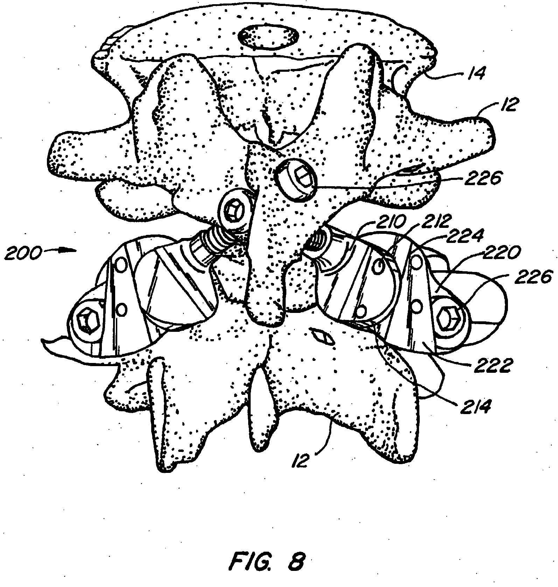

Another implantable arthroplasty device ***200*** is illustrated in **FIG. 8**. **FIG. 8** shows an artificial joint structure for replacing a natural facet joint **(****FIG. 3**, ***32***). The cephalad structure ***210*** has a bearing element ***212*** with a bearing surface ***214.*** The caudal structure ***220*** has a bearing element ***222*** with a bearing surface ***224.*** Conventional fixation elements ***226*** attach the cephalad structure ***210*** and caudal structure ***220*** to a vertebra ***14*** in an orientation and position that places bearing surface ***214*** in approximately the same location as the natural facet joint surface the caudal facet joint ***220*** replaces. As will be appreciated by those of skill in the art, the facet joint may also be placed in a location other than the natural facet joint location.

The cephalad structure ***210*** and the caudal structure ***220*** illustrated in **FIG. 8** address issues relating to facet joint degeneration and can restore biomechanical motion. The illustrated structures can also be configured to provide design features having more modular components or to provide attaching mechanism for attachment to the spinal bone in a variety of orientations and/or locations.

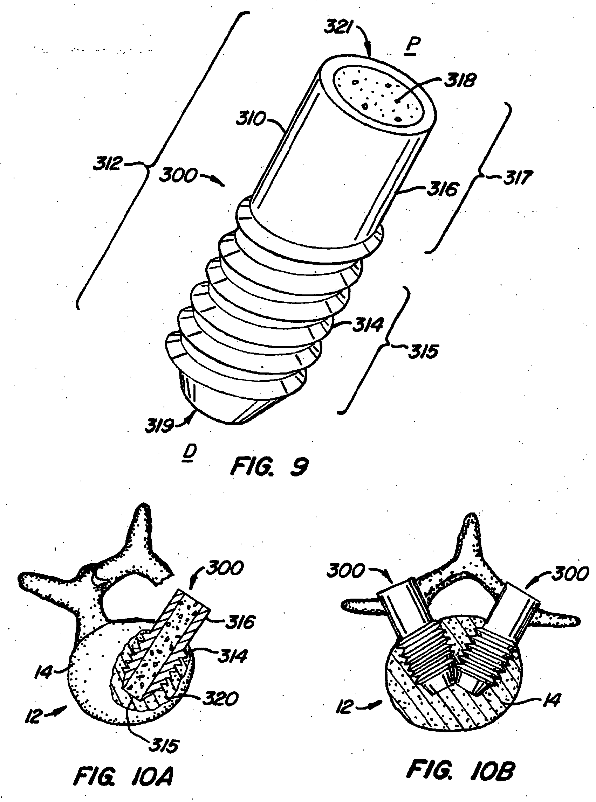

Turning now to **FIG. 9**, an implantable device suitable for spine reconstruction is configured. The spine reconstruction device *300* comprises an implantable restoration unit designed to compensate for natural spinal anatomy that has been damaged by surgical procedures, such as drilling holes, is damaged (due to trauma, tumor and/or removal of spinal structures and/or spinal instrumentation) or is missing, *e.g*. missing pedicles, posterior arch, etc. As a result of damage to the spinal anatomy there may not be enough natural bone structure available to enable the spine anatomy to be modified or restored using implantable devices, such as those described above. Thus, one or more implantable restoration units can be used to augment the spinal anatomy enabling use, or continued use, of an implantable device. The implantable restoration unit ***300*** is comprised of an elongated tube ***310*** sized to engage a portion of a vertebral body having a proximal end ***p*** and a distal end ***d**.* The elongated tube ***310*** has a length ***312*** that can be modified to provide an anchoring feature, such as threads ***314*** (illustrated), and/or to provide a surface features that assists in bony in-growth. Augmentation can be provided using, for example, resorbable bone cement, which increases the strength of both cannulated and non-cannulated restoration units. The anchoring feature is positioned distally ***315*** on the device such that it is positioned further within the body relative to the opposing end. Where the elongated tube ***310*** extends beyond the exterior of the vertebral body, the exterior surface of the tube ***310*** can be smooth ***316.*** The smooth exterior surface is positioned proximally ***317*** on the device. At least a portion of the interior of the tube can be filled with bone filler or allograft material ***318.*** Suitable bone filler material includes, the use of bone material derived from demineralized allogenic or xenogenic bone and can contain substances for example, bone morphogenic protein, which induce bone regeneration at a defect site. *See,* U.S. Patent Nos. 5,405,390; 5,314,476; 5,284,655; 5,510,396; 4,394,370; and 4,472,840, which disclose compositions containing demineralized bone powder. *See also* U.S. Patent No. 6,340,477, which discloses a bone matrix composition. The distal end ***315*** of the device ***300*** can feature an aperture ***319*** thus enabling the allograft material ***318*** to come into contact with natural bone material within an area to be restored, for example, a vertebral body. Alternatively, a hardenable material can be provided within the device ***300.*** The hardenable material can comprise bone cement, such as polymethyl methacrylate, or any of a variety of suitable biocompatible polymers known to those skilled in the art. As will be appreciated by those skilled in the art, the amount of bone filler used in conjunction with the restoration device ***300*** can vary. Thus, for example, the entire lumen of the device can be filled with bone filler, or only a portion of the device. In addition to the aperture ***319*** provided at the distal end ***315,*** additional apertures can be provided along the length of the elongated body to enable the content located within the device to contact the bone. As shown in **FIG. 9**, the restoration device ***300*** has been configured with a flat proximal end ***321.*** The flat end would be useful where the restoration device ***300*** is implanted in a location where the natural bone anatomy is flat, or substantially flat. Alternatively, a flat configuration could be useful where the design of the device to be mated with the restoration device has been configured to be joined to a flat surface. Alternatively, as will be appreciated by those skilled in the art, the proximal end ***321*** of the restoration device ***300*** can be configured such that the end is adapted to conform to the anatomy of the bone structure (or to the design of the spinal implant) to allow anatomical restoration (or allow implantation of the implant).

As shown in **FIG. 10A****,** the implantable restoration unit ***300*** has been implanted within a vertebral body ***14.*** The threads ***314*** located on the distal end ***315*** are positioned within the vertebral body ***14*.** The smooth end ***316*** located at the proximal end ***321*** is positioned outside the vertebral body ***14*** such that the implantable restoration unit ***300*** would substitute for a pedicle (*see* **FIG. 2****,** ***6*****).** Once the implantable restoration unit is in place, a replacement device can be provided, such as a device that replaces the transverse process. Bone cement ***320,*** or other suitable material, can be used to further anchor the device *300* within the target bone. Turning now to **FIG. 10B****,** a spine segment having both its pedicles ***16, 16'** (shown in* ***Fig. 2****)* replaced with implantable restoration unit ***300*** devices is depicted. In addition to be used to replace, restore and/or augment the pedicles, the implantable restoration unit can be used to replace, restore and/or augment the lamina and any of the processes of the vertebral body.

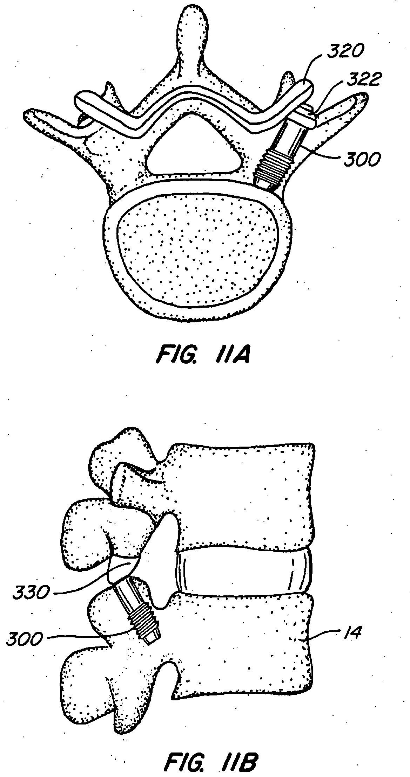

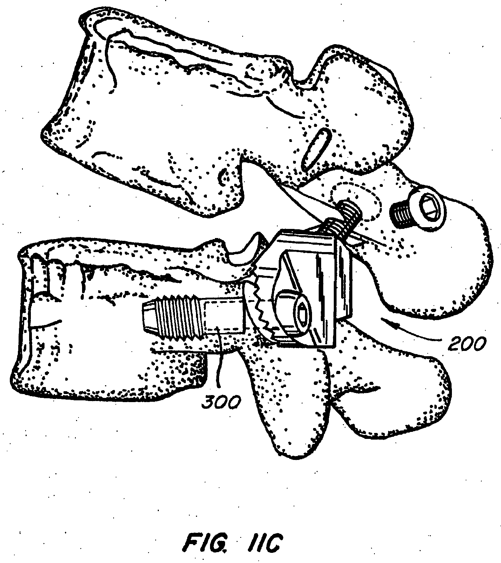

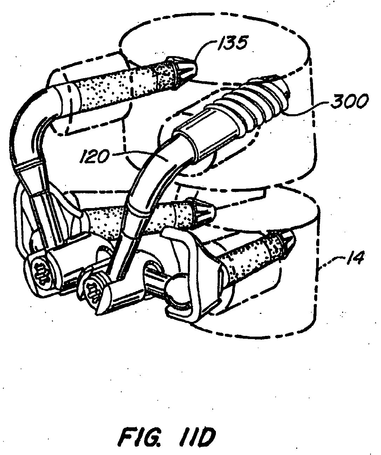

**FIG. 11A** illustrates a implantable restoration unit ***300*** as described above with respect to **FIG. 9** implanted within in a vertebral body ***14*** in combination with a spinal arthroplasty device ***320.*** The device ***300*** has been implanted such that it is positioned to substitute for a pedicle (***16*** of **FIG. 2**) and provides a replacement pedicle surface ***322*** for the arthroplasty device ***320*** to engage. The arthroplasty device ***320*** can be configured to engage the pedicle or replacement pedicle surface provided during the surgical or revision surgical procedure. The device ***320*** can further be configured to provide an anchoring mechanism, such as a screw or bolt, which passes through an aperture or interior bore provided in the device ***320*** and screws into the implantable restoration unit ***300.*** **FIG. 11B** illustrates a yet another implantable restoration unit ***300*** implanted with a facet repair device ***330.*** The implantable restoration unit ***300*** is positioned within the vertebral body ***14*** at its distal end and engages the facet repair device ***330*** at its proximal end. In yet another example of the versatile implantable restoration unit ***300***, **FIG. 11C** illustrates an implantable restoration unit ***300*** implanted with a device for replacing resected facet joints. The implantable restoration unit ***300*** is positioned to provide a surface for the facet joint device to engage. As is evident from these examples, the implantable restoration unit ***300*** can be used in a wide variety of applications and in combination with a wide variety of implantable devices. **FIG. 11D** illustrates an embodiment of the invention, wherein the implantable restoration unit ***300*** is used in conjunction with the arthroplasty device of **FIG. 7** to provide a surface for a cephalad arm to mate with.

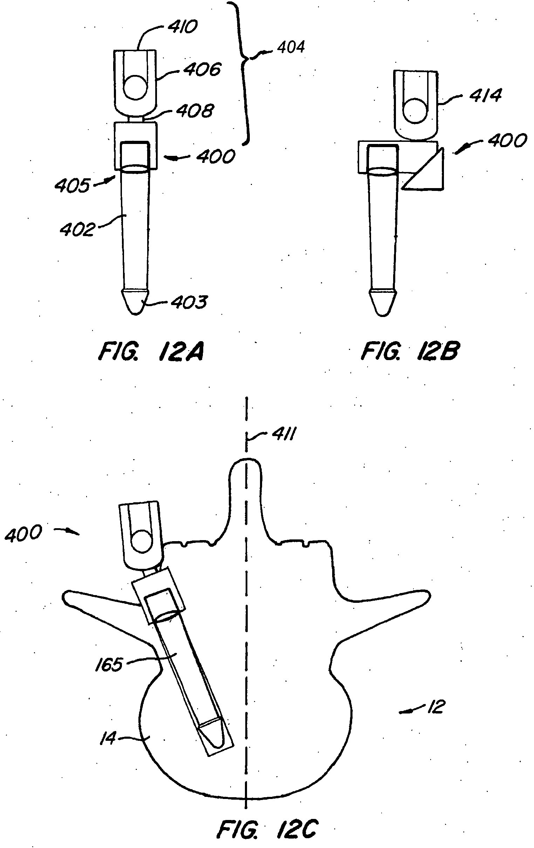

**FIG.12A** illustrates an adapter cap device ***400*** for revising an implanted arthroplasty device. The adapter cap device ***400*** is designed to receive a portion of an implanted spinal arthroplasty device, such as those illustrated above. The device ***400*** is adapted to attach to an elongated shaft or stem ***402*** which has been positioned within a vertebral body 12 of a spine. The stem ***402*** can be, *e.g.,* the previously implanted shaft of an existing arthroplasty device (such as that shown in **FIG. 7**) and a pointed distal end ***403*.** The stem ***402*** is configured such that it can have a smooth exterior or an exterior surface treatment (as illustrated). The exterior treatment would be provided to facilitate the mating of the stem of the adapter cap device to the body with which it was mating. The proximal end ***403*** of device ***400*** has an adapter cap ***404*** which has been configured such that it can be attached to and removed from the stem ***402**.* The adapter cap ***404*** is configured to provide an aperture sized with a diameter that enables a snug fit around the diameter of the shaft ***402**.* The adapter cap ***404*** can be joined to the stem ***402*** using methods known in the art, including, but not limited to, swaging, crimping and/or bonding. The adapter cap ***404*** is connected to a polyaxial housing ***406*** by a neck ***408**.* The neck ***408*** connection enables the polyaxial housing ***406*** to assume differing orientations relative to the orientation of the stem ***402***, thus accommodating a variety of arthroplasty devices. The proximal end ***410*** of the polyaxial housing can be configured to receive a variety of connectors and devices depending upon the design of the arthroplasty device to be accommodated. Alternatively, the existing proximal end of an implanted device may be cut and removed, and the adapter cap device ***400*** may be positioned over the remaining neck of the device.

Turning now to **FIG. 12B****,** a revision polyaxial device ***400*** similar to the device of **FIG. 12A** is depicted. The shaft ***402*** mates with the adapter cap ***414*** to provide an arthroplasty device receiving housing that is offset to a central axis ***x*** of the stem ***402**.* The adapter cap ***414*** is similarly configured to the adapter cap ***404*** of **FIG. 12A** in that the cap portion enables the housing ***406*** to be positioned off the central axis *x* of the stem. The offset adapter cap provides additional flexibility to the design enabling the adapter cap to mate with a variety of anatomical surfaces, including resected surfaces or damaged surfaces, along with a wide variety of arthroplasty devices. The cap attaches to stem ***165***.

**FIG. 12C** illustrates a device ***400*** implanted into a vertebral body ***12*** such that the cap of the device can function as a pedicle replacement. In this illustration, there is no preexisting stem or other device, and device ***400*** therefore includes an integral stem ***165***. The device ***400*** can be implanted such that the device (desirably and generally) does not intersect the central sagittal axis ***411*** of the vertebral body. The housing and cap can be configured to provide a fixed structure, or can be configured to enable the housing and cap to be engaged such that rotation between the two elements is enabled.

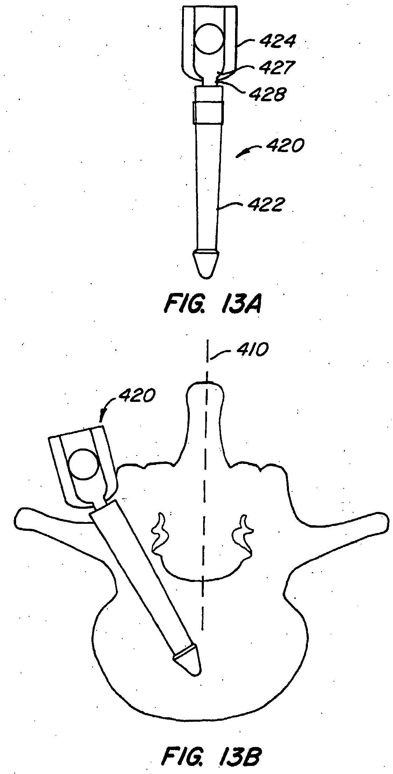

**FIG. 13A** illustrates another configuration of a device ***420*** of **FIG**. **12** suitable for a stand-alone application, *i.e.,* without attaching to a portion of an existing implant. The stem ***422*** is configured such that it incorporates a polyaxial element or housing ***424.*** The housing ***424*** further engages a ball ***427*** attached to a neck ***428*** that provides further flexibility between the polyaxial element and an arthroplasty device to which it is mated. When implanted, as shown in **FIG. 13B****,** the device ***420*** can be implanted into a vertebral body ***12*** such that the device abuts or intersects the central sagittal axis ***410*** of the vertebral body. These devices can be preformed and used for implantation during the installation of an initial arthroplasty device or can be used in conjunction with revision surgery to provide additional flexibility and adaptability to the device performance. In addition, these devices can be used to attach to commercially available spinal fusion instrumentation, including generally-available spine rods and

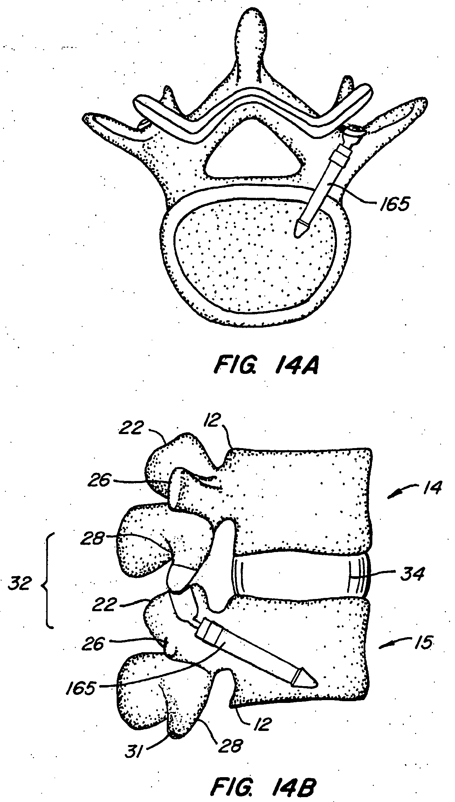

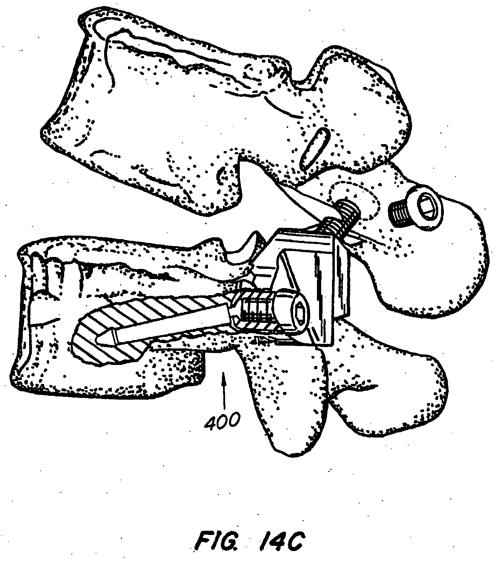

**FIG. 14A** illustrates an adapter cap implanted in combination with an arthroplasty device. The arthroplasty device comprises a body that engages a surface of the vertebra and a stem ***165**.* The adapter cap of **FIG**. **13** has been modified to enable the adapter to communicate with the arthroplasty device. **FIG. 14B** illustrates an adapter cap implanted in combination with another arthroplasty device. The adapter cap has again been modified to enable the cap to mate with the implantable device that restores the inferior facet. Turning now to **FIG. 14C** yet another adapter cap assembly ***400*** is depicted implanted in combination with the facet replacement device of **FIG**. **8**. In this instance, the adapter cap has been modified to enable the device to receive the threaded bolt that secures the device.

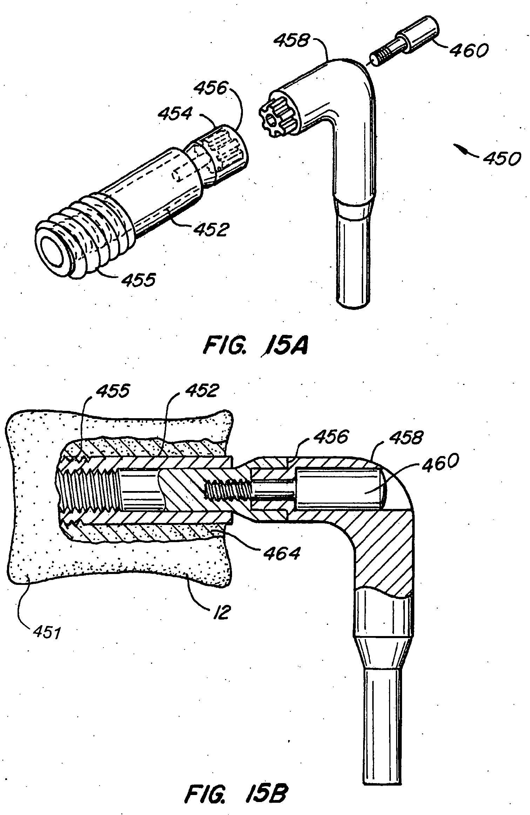

**FIG. 15A** illustrates a perspective view of component parts of a replaceable modular stem system for use in an implantable spinal arthroplasty device. The system ***450*** includes an internally and externally threaded auxiliary sleeve shell ***452**.* A threaded female tip ***454*** fits within an aperture of the sleeve shell ***452.*** The threaded female tip ***454*** has a threaded first end ***455*** and a configured aperture ***456*** on the opposing end. A male stem ***458*** is provided for communicating with the female threaded tip ***454.*** The male stem ***458*** has a configured protrusion ***460*** (male member) on one end that mates with the configured aperture ***456*** (female member) on the threaded female tip ***454.*** The configured protrusion ***460*** is configured to key within the female threaded tip ***454*** to create a snug fit between the configured aperture ***456*** and the configured protrusion ***460*.** The system ***450*** can be further held together by use of a threaded dowel pin ***460,*** that fits within a receiving aperture ***462*** of the stem ***458.*** Once the system is configured and in place, the snug fit and keyed configuration of the threaded tip ***454*** and configured aperture ***456*** for receiving the tip prevents movement of the stem ***458*** with respect to the sleeve shell ***452*.** The threaded dowel pin ***460,*** further prevents movement of the stem ***458*** with respect to the sleeve shell ***452*** and the system overall. If desired, the stems ***458*** can comprise a set of differing size, length and/or shape stems to accommodate anatomical and/or surgical variability, as previously described.

Turning now to **FIG. 15B****,** a side view of replaceable modular stem system ***450*** is illustrated. As can be appreciated by this view, the sleeve shell ***452*** can be threaded into target bone ***451*** using the external threads ***455.*** Anchoring of the sleeve shell ***452*** to the bone ***451*** can be achieved by use of the threads alone (*e.g*., for healthy bone) or the threads in combination with bone cement ***464***. Additionally, where the target bone is weak, the site can be drilled-out, bone cement can be applied, and the stem can then be threaded into the bone cement. The revision system depicted in **FIGS. 15A-B** can be used to revise or replace, for example, caudal and/or cephalad arms of a spinal arthroplasty device, such as the devices depicted in **FIGS. 6-7**. Due to the modularity of the design, substitutes for components can easily be employed. As described with respect to **FIG. 9**, the sleeve shell ***452*** can be used to replace a missing or damaged pedicle, and can be used to assist in reconstruction.

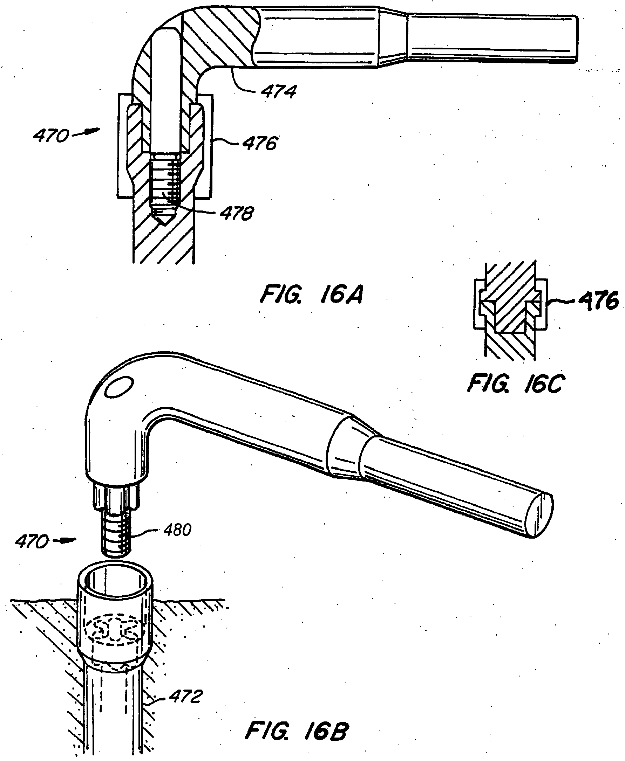

**FIG 16A** illustrates a side view of a replaceable modular stem system ***470*** of an alternate configuration to the system illustrated in **FIG. 15**. The modular stem system ***470*** has a sleeve feature ***472*** that enables the male stem ***474*** to fit within the female stem ***472.*** Additionally, an external tie-down or sleeve ***476*** can be provided that provides an additional anchoring feature between the male and female stem. An alternate anchoring mechanism, in the form of a set screw ***478*,** is provided to further anchor the two pieces together. The set screw can have a configured aperture on its upper surface shaped to mate with a corresponding driver tool, *e.g*. cross-headed screwdriver, flat headed screw driver, and the like. **FIG. 16B** illustrates the modular system depicted in **FIG. 16A****,** further illustrating the configured aperture ***486*** on the female stem ***472*** and the configured male protrusion ***480*** on the male stem that provides an anti-rotation and/or anti-displacement feature(s) between the two pieces when mated. **FIG. 16C** illustrates the external tie-down ***476*** of **FIG**. **16A****.**

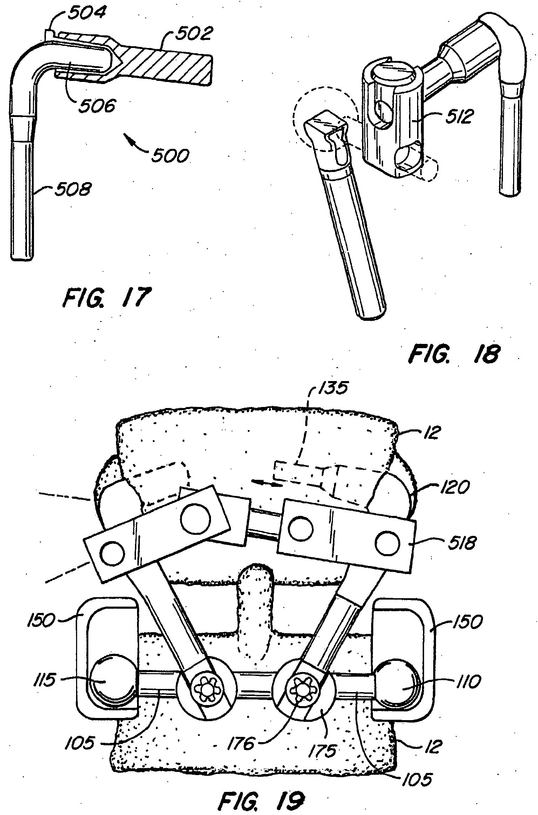

**FIG. 17** illustrates yet another alternate modular stem system ***500*** having a modular stem ***502*** with a female opening ***504*** for receiving a male connector ***506*** of a connecting arm ***508**.* The mating of the modular stem ***502*** and the connecting arm ***508*** is further enhanced by virtue of a friction and/or compression fit between the two components. Friction fit can be achieved by use of, for example, a bushing ***510**.* A suitable bushing would include a swaging bushing. Systems of this design can be used on a post-operative revision of a total facet arthroplasty device. Where the total facet arthroplasty device is revised, the surgeon may cut the cephalad stem of the total facet arthroplasty device and remove the caudal bearing. A suitable cut would be made perpendicular to the axis of the exposed shaft. Following posterior lumbar interbody fusion (PLIF) or translaminar PLIF, a modular rod extension can be swaged into a dovetail feature, as shown in **FIG. 18**. **FIG. 18** further illustrates the modular stem system of **FIG**. **17** connected to a housing ***512.*** The housing can be used to link elements, such as the cephalad element to the caudal element, or to connect cephalad arms to a cross-member. If desired, the caudal cup can be removed from the caudal stem, to allow access to the intervertebral space for a PLIF, with the caudal cup or some other construct replaced back onto the caudal stem once the interbody procedure has been completed.

As shown in **FIG. 19** an implanted arthroplasty device can have a cross-linking arm ***518*** installed to further reinforce the assembly. The cross-linking arm can also further prevent movement of the arms relative to the cross-bar member. The cross-linking arm ***518*** can be implanted to further reinforce the device or provide additional control of movement or articulation between the arms, e.g. cephalad arms, and the cross-member, or can be used to secure a single loose cephalad arm in a desired position and/or orientation. The cross-linking arm can be formed of a single device that connects at either end, using any suitable connection mechanism or structure. Alternatively, the cross-linking arm can be comprised of components that mate, to functionally achieve an arm and provide additional flexibility with respect to length.

**FIG. 20A** illustrates a securing device for use in connection with an arthroplasty device to revise and/or modify, control, or limit motion of the arthroplasty device. The securing device has a body ***520*** with a distal surface ***521*** having pair of prongs ***522, 522'*.** When installed, the prongs ***522, 522'*** form a base and are positioned below the crossbar member and the indenture ***524*** of the securing device engages the anchors on three sides. When used with a device of **FIG**. 7, the prongs can be positioned below the caudal cup which receives an end of the crossbar member, while the top sits above the crossbar end (***110***, ***115***) to secure the end in place within the caudal cup ***150.***

The prongs ***522, 522'*** engage a wall ***526*** of the securing device on one side. The wall ***526*** mates with a top or roof ***528*** that fits above the cross-bar member. The top ***528*** has an aperture ***529.*** The aperture ***529*** can function as a detent, catch or plunger to snap fit over the ball end ***110*** of the crossbar member in an arthroplasty device. Alternatively, the securing device can be a securing mechanism, such as a set screw ***530*.** **FIG. 20B** is a top view of the securing device ***520.*** From this perspective, it is apparent that the top ***528*** can be positioned off a central axis of the device to the two prongs ***522, 522',*** thus also potentially positioning the aperture ***529*** off the central axis as well. **FIG. 20C** is a side view of the securing device, illustrating the angled configurations of the sides ***531, 531'*** back wall *526.* The angled configuration positions the top ***528,*** which can have a smaller dimension in at least one direction (*e.g.*, length or width) than the length or width formed by the prongs and the wall. **FIG. 20D** is a bottom view of the securing device ***520.*** **FIG. 20E** is a cross-sectional view of the securing device taken through an axis parallel to the prongs ***522, 522'.***

**FIG. 21A** illustrates a side view of the securing device of **FIG**. **21** in combination with a portion of an arthroplasty device, such as the arthroplasty device of **FIG. 7**. The prongs ***522, 522'*** si**t** below the caudal cup ***150***, holding the caudal cup in a fixed position. The top ***528*** of the securing device ***520*** sits above an end of the cross-member ***110**,* which fits within the caudal cup ***150.*** An anchoring device ***530*** (see **FIG. 20A**) can be fed through the aperture to engage the end of the cross-member and hold it in position within the caudal cup ***150.*** As illustrated, the caudal cup ***150*** is tilted ***t*** toward an axial plane ***52,*** enabling the caudal cup to secure the cross-member at a location. Adjustment of the position of the caudal cup relative to the cross-member end can affect the position of the device. **FIG. 21B** illustrates a perspective view of the securing device in combination with a portion of the arthroplasty device. From this perspective, a set screw ***530*** located within the aperture ***529*** on the top of the securing device can be seen. **FIG. 21C** is a perspective view from a partially anterior view of the securing device again in combination with a portion of the arthroplasty device. **FIG. 21D** is a top view of the securing device ***520*** with a portion of the arthroplasty device. As evident from this perspective, the caudal cup extends on one side past the prong ***522'.*** The set screw ***530*** is positioned off-center relative to the length of the securing device, but the top of the securing device is positioned over the end of the cross-member. **FIG. 21E** is a bottom view of the securing device engaging an arthroplasty device. From this view, it is illustrated that the prongs ***522, 522'*** are seated beneath, for example, the caudal cup of the arthroplasty device.

Thus, the implanted arthroplasty device can be revised to incorporate locks or "fusion caps" that desirably convert the device from an articulating joint replacement construct to a non-articulating (or controlled and/or limited articulation) spinal fusion construct. The fusion cap can be installed on or into the caudal cups to desirably immobilize the cephalad bearings within the cups. The fusion caps could immobilize the cephalad bearings by direct compression or contact, through use of a set screw or other device to secure the cephalad bearing relative to the cup, or the fusion cap could contain or cover an encapsulating material, such as bone cement, which could fill the caudal cup and immobilize the cephalad bearing. Various techniques could be used in conjunction with the installation of such fusion caps, and the cap could be installed prior to, during, or after the completion of a concurrent spinal fusion procedure, including the removal of intervertebral disc material, installation of fusion cages, and/or introduction of material (such as bone graft material) that desirably promotes spinal fusion. Alternative configurations could incorporate bearings of different shapes or sizes (not shown), including square or non-spherical bearings and/or bearings shaped to that fit snugly into and accommodate most or all of the interior of the caudal cup (not shown), that can be secured within the cup in a similar manner.

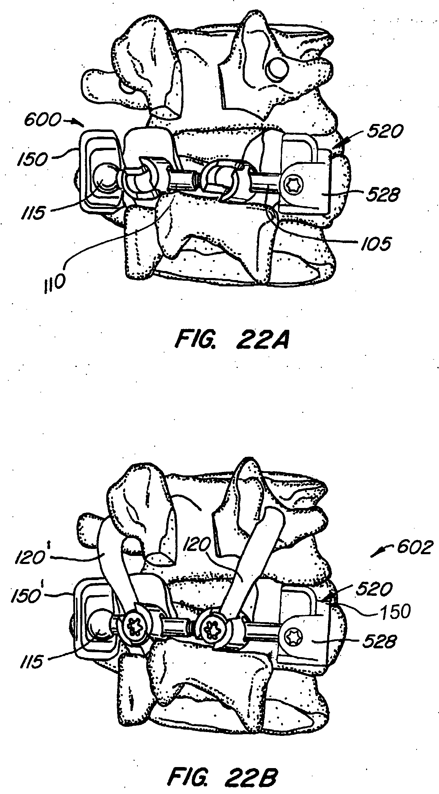

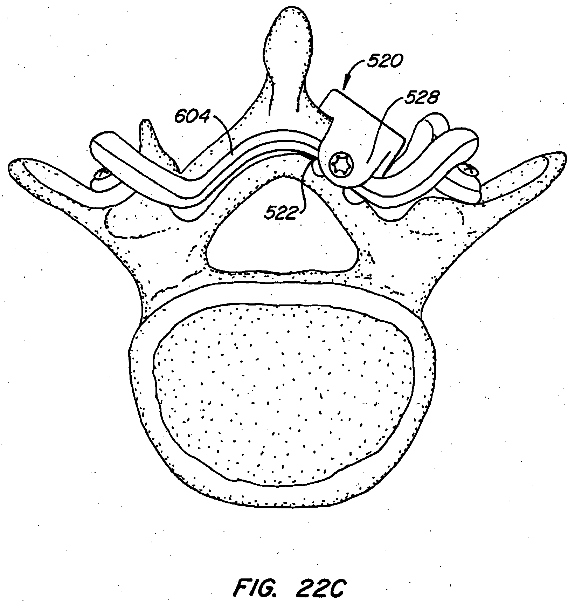

Turning now to **FIG. 22A****,** a perspective view of an implanted arthroplasty device ***600*** with the securing device of **FIG. 21** is illustrated. The arthroplasty device ***600*** features a pair of caudal cups ***150*** engaging a cross-member ***110.*** The cephalad arms have been removed, but it has been determined desirable to keep the caudal cups and cross-bar in place. The use of the securing device enables the caudal cup and crossbar member to be retained in position even without one or more of the cephalad arms to anchor the cross-member. Additionally, as will be appreciated by those of skill in the art, one of the two cephalad arms could be removed with the use of one or two of the securing devices to provide a three-point secured device (i.e., rigidly connecting two caudal cups to a single cephalad arm). The securing device engages the caudal cup and an end of the cross-member in the manner described above. **FIG. 22B** is a perspective view of another implanted arthroplasty device ***602*** having a pair of caudal cups ***150, 150'*** engaging a cross-member ***110*** and a pair of cephalad arms ***120, 120'*** extending vertically toward the adjacent vertebra ***12*** along with the securing device of **FIG. 21****.** **FIG. 22C** is a perspective view of yet another implanted arthroplasty device ***604*** with the securing device of **FIG**. **21**.

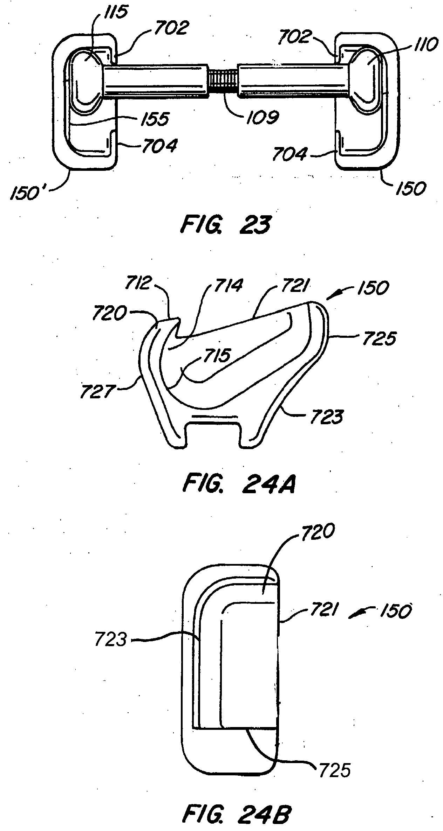

Additional modifications of the caudal cup of an arthroplasty device are also possible in order to improve the operation and reliability of the arthroplasty device through the range of spinal motion. Further, these modifications can change the operation of the device from one enabling a full range of motion, to a device that enables less than a full range of motion, or to a device that restricts range of motion (this "restriction" could extend from allowing full motion to allowing partial or controlled motion to allowing no motion - thus functionally achieving some of all of the effects of a fusion device). One such modification is illustrated in **FIG. 23**. Caudal cup ***150'*** is a modified version of the caudal cup ***150*** shown in **FIG. 7**. The caudal cup ***150'*** includes an upper crossbar end retainer ***702*** and a lower crossbar end retainer ***704.*** The upper and lower crossbar end retainers ***702*, *704*** may optionally be provided to reduce the likelihood that the crossbar ends ***110, 115*** will slide out of contact with or leave an acceptable area adjacent the caudal cup surface ***155*** (dislocate). In a similar manner, the posterior surface of the caudal cup could also be closed (not shown), thereby capturing and holding the crossbar ends ***110, 115*** and limiting and/or preventing posterior movement of the crossbar relative to the caudal cups. In this alternate device, the caudal cups could also comprise a "clamshell" design with the lower portion (as shown in **FIG. 23**) and a mating shape (not shown) that clamps, bolts, clips, or bonds to the lower portion, substantially closing the posterior side of the cup.

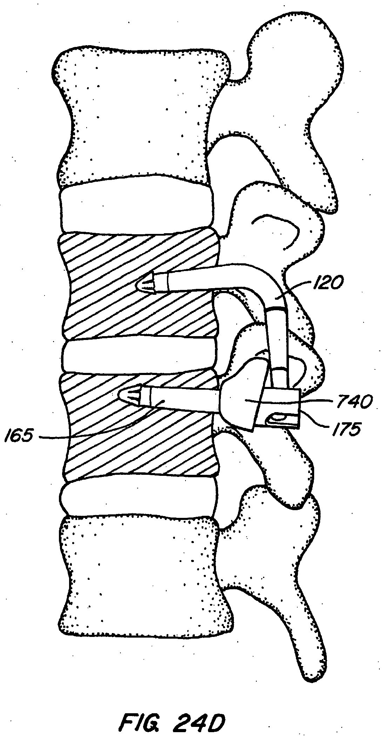

**FIG. 24A** illustrates yet another alternate design of a caudal cup ***150*** incorporating a flange ***712*** which creates a pocket ***714*** to contain and/or secure a cephalad bearing element of an arthroplasty device (such as the device shown in **FIG. 6**) that can be positioned within the pocket when the arthroplasty device is assembled. When this design of caudal cup ***150*** is deployed in an arthroplasty device, it secures the bearing element ***115** (as shown in* **FIG. 23**) when the arthroplasty device is articulated to one or more extreme limits of its range of motion. Thus, for example, when the cephalad and caudal elements are compressed together (such as during extension of the spine), the cephalad bearing element (***115*** in **FIG. 23**) will slide along the caudal bearing element in the cephalad direction, coming to rest in the pocket ***714*** formed by the interior surface ***715*** of the caudal cup ***150.*** When the bearing (not shown) is positioned within the pocket ***714*** any increased compressive force acting on the device will desirably seat the bearing even further into the pocket ***714,*** reducing and/or eliminating any opportunity for the bearing to slide out of the cup and potentially dislocate the device. If desired, a similar flange and pocket (not shown) can be formed on the opposing (cephalad) side of the caudal cup ***150,*** to capture the cephalad bearing and prevent dislocation of the bearing surface from the cephalad cut during flexion of the device. Thus, the flange can provide a hard stop for the bearing surface during flexion. If desired, this alternative caudal cup design (incorporating the flange 712) could be implanted in patients prone to dislocation (during the initial facet replacement procedure) or it could be implanted in a subsequent surgical procedure to replace a non-flanged caudal cup after the patient has dislocated the facet joint replacement incorporating a non-flanged cup design. Similarly, the various other devices disclosed herein could be used to replace and/or retrofit components already implanted within a patient, or could be used prophylactically during the initial implantation surgery to ensure against failure of the implant.

In alternative devices, such as that shown in **FIG. 24B****,** additional crossbar motion can be accommodated by altering the caudal cup width (w_{cup}) or adjusting the distance between the medial edge 721 and the lateral edge 723 in some devices. If desired, the upper edge ***720*** can be configured to curve over the top to enclose (either partially or fully) the upper portion of the cup ***150*.** The radius of the curve that transitions between the lateral edge ***723*** and the upper edge ***720*** and the radius of the curve that transitions between the lateral edge ***723*** and the lower edge ***725*** may also be adjusted to accommodate the various shapes of the crossbar end ***115*** and outer surface. The medial edge ***721*** and lateral edge ***723*** can be configured such that the edges are nonparallel, with respect to each other. The medial edge ***721*** and the lateral edge ***723*** could have an arcuate shape, or the cup ***150*** could be completely enclosed with a flexible and/or rigid cover or cap. In other embodiments, the medial edge ***721*** could have a raised lip or ridge (not shown) which would desirably assist in retaining the cephalad bearing within the caudal cup ***150**.* Such arrangements could help prevent dislocation of the construct and/or allow for spontaneous and/or controlled relocation of the bearing surface (operatively, minimally invasively or non-invasively, including non-operative manipulation of the patient's spine through chiropractic procedures, etc.).

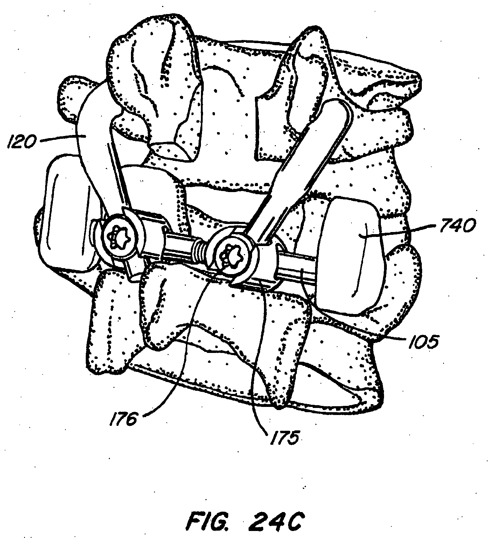

In one alternate device, once the cephalad and caudal components of the device has been secured to the targeted vertebral bodies, one or more elastic compression devices or "bands" ***740*** could be secured about the caudal cups and bearing elements (see **FIG. 24C****),** to the vertebral bodies themselves, between other parts of the cephalad or caudal arms, or any combination thereof. This configuration could be especially useful if a device dislocates one or more times. Properly positioned and/or tensioned, these "bands" would tend to keep the bearing surfaces and caudal cups in contact and/or close proximity, even under extreme and/or unusual loading conditions, and thus reduce and/or eliminate the opportunity for the bearing elements to dislocate. Moreover, in the event that dislocation of the implant did occur, the bands could prevent and/or limit motion of the dislocated joint (by holding the bearing surfaces and caudal cups together), and thus reduce or eliminate damage to other tissues (such as the spinal cord, various other nerves and/or circulatory/connective tissues) resulting from the dislocation. In fact, the compression of the bands might make it possible to eventually "reduce" the dislocation and/or repair the dislocated device through external manipulation and/or minimally-invasive surgery. If desired, one or more "bands" could be secured between the articulating surfaces of the device, or between the various arms, cups, stems and/or cross-arms of the construct elements, with varying results. In one device, such a band could be looped around the base of the caudal cup, and around the corresponding cross-arm, in a figure-8 shape. Properly positioned and tensioned, this arrangement would allow the cup and cephalad bearing to articulate without allowing the band to slip off (either or both) the cup and cross-arm. Depending upon the length and size of the band, and the tension therein, the band could positioned and tighten to reduce and/or ultimately prevent any significant articulation of one or both sides of the facet joint replacement device. If desired, the band could be tightened and/or loosened in a minimally-invasive manner, during the implantation procedure and/or during a subsequent procedure.

In another alternate device, the compression device could comprise an elastic or pliable material, which may or may not be surrounded by a non-elastic housing, whereby the elastic material allows various movement of the bearing surfaces (with resistance commensurate to the flexibility of the material, as well as flexibility allowed by the coupling to the device components), but the optional non-elastic housing acts as an ultimate "stop" to movement of the bearing surfaces relative to the caudal cup. Such device could include one or more "encapsulated" bearing surfaces, such as shown in **FIGS. 24C-D****,** which show two caudal cup and cephalad bearing pairs (of a facet replacement device), each pair surrounded by a flexible skin or "jacket" ***740*** which permits relative movement between the cup and bearing, but which desirably encapsulates or isolates the cup and bearing pair from the surrounding environment (totally or partially or some combination thereof). In practice, the jacket ***740*** can serve many functions, including (but not limited to) (1) as a shock absorber or brake to slow, control, modify and/or limit movement of the bearing/cup complex throughout and/or at the extreme ranges of motion, (2) as a stop or limiter to reduce and/or prevent complete or partial dislocation of the joint, (3) as a barrier to prevent surrounding tissues from invading the bearing surfaces and/or being "pinched" or damaged between moving surfaces, and (4) as a barrier or "filter" to prevent "bearing wear particulate," or other bearing by-products, from reaching and impacting surrounding tissues (or to contain fluids or other materials including, but not limited to, joint lubricating fluids, antibiotics and/or fluids that provide a biological marker and/or indicator - including bioreactive materials - upon rupture or compromise of the barrier). In a similar manner, the jacket ***740*** could encompass the entire bearing construct, with only the cephalad and caudal stems (and possibly the crossbar, depending upon whether the jacket encompasses one or both bearing constructs) protruding through the jacket and extending into the vertebral bodies. Depending upon the type of polymer (or other material) used, as well as the physical properties and orientation of the polymer, the jacket ***740*** could be designed to control the motion of the device in a desired manner, and could also control the movement of the device to more accurately replicate the natural motion of the spinal segment. For instance, a polymer jacket could be designed to allow a greater degree of freedom in flexion/extension, but limit (to some extent) the degree of lateral bending or torsion of the same segment, by proper choice and orientation of the polymer or other material. The band could comprise a flexible, polymeric (including, but not limited to, biocompatible polymeric) material.

The physical properties of the materials used could be selected based on an ability to alter over time or in response to one or more biological, environmental, temperature and/or externally induced factors, altering the properties of the material (*i.e*., polymers, ceramics, metals - Nitinol - etc.). For example, the material could comprise a material that hardens over time (or in the presence of body fluids, proteins, or body heat, etc.), which initially allows the components to freely articulate at the time of implantation (and thus minimize the stresses experienced by the anchoring components), but which hardens and subsequently resists movement to a greater degree once the component anchoring has solidified or bonded to the surrounding bone. Biodegradable materials can be used to adapt an implanted device to achieve a temporary fixation of the device. By preventing movement for a period of time, healing can be facilitated, among other things. Once a suitable amount of time has passed and the biodegradable material (or other types of materials) degrades (or otherwise alters its material properties in some manner), the device will then return to its initial state of biomechanical movement. Where the material alteration is induced by externally induced factors (such as directed radiation, rf and/or sonic energy), the external factor could desirably alter the physical properties of the material(s) in a partially or completely reversible manner (depending upon the type, duration, frequency and/or amplitude of the induced factor), allowing for controlled alteration of the material properties in a minimally-invasive and/or completely non-invasive manner.

Similarly, the "band" could comprise an elastic, non-elastic or rigid material, such as stainless steel cable, which desirably prevents relative motion of the device components beyond a certain pre-defined maximum extension, flexion, rotation and/or torsion. The band could alternatively be installed to limit motion of the device to prevent dislocation, or to minimize or control the articulation of the device to some degree (such as to protect a disc replacement device against unwanted motion in one or more directions, protect an adjacent fused level against unwanted stresses, or to protect various tissues from experiencing stresses and/or damage). If desired, the cable could be tightened or loosened post-surgery, in a minimally-invasive manner, to alter performance of the device.

Where the revision involves a spinal level incorporating an artificial disc replacement, the revision instrumentation could include wedges or "shims" that could be used to immobilize the artificial disc (thereby augmenting the motion modification provided by the revision component) and/or further increase the likelihood of achieving a successful fusion in combination with the revision constructs described herein.

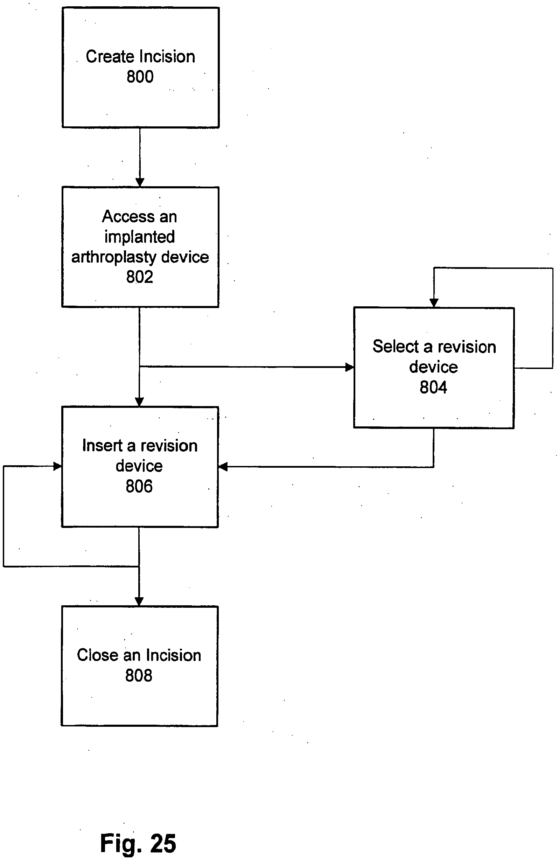

**FIG. 25** illustrates a method for altering the biomechanics of an implanted spinal arthroplasty device, or revising an implanted arthroplasty device. An incision may be created in a selected location to access the implanted arthroplasty device ***800**.* However, as will be appreciated by those of skill in the art, scenarios can arise wherein a surgeon is implanting a spinal arthroplasty device and determines that the condition of the patient warrants revising the biomechanics of the device during the initial implantation. More commonly, however, revision will be performed at a time subsequent to implantation of the spinal arthroplasty device, thus requiring a second, or subsequent, surgical intervention.

Once the incision is made the implanted arthroplasty device is accessed ***802.*** Unless, the biomechanics have been assessed prior to surgical access and a device has been preselected, the surgeon can then select a revision device ***804**, e.g.* from a kit, that is adapted to alter the biomechanics or revise the arthroplasty device. Whether selected in advance of surgery, or during the surgical procedure, the surgeon next inserts the revision device ***806**.* As discussed above, the revision device can be one that alters the biomechanics, whether temporarily or permanently, or otherwise revises the implanted arthroplasty device. The step of selecting a revision device and implanting a revision device can be repeated as often as required to achieve the desired result. Once the surgeon is satisfied that the desired result is achieved, the incision is closed ***808**.*

While preferred embodiments of the present invention have been shown and described herein, it will be obvious to those skilled in the art that such embodiments are provided by way of example only. Numerous variations, changes, and substitutions will now occur to those skilled in the art. The scope of the invention is defined by the claims.

## Claims

1. A spinal facet joint prosthesis (100) comprising:
a crossbar (105),
a first and a second cephalad arms (120,120') configured for implantation in conjunction with a first vertebral body (14), and
a pair of caudal arms (150, 150') configured for implantation in conjunction with a second vertebral body (14');
wherein the cooperative operation of the crossbar (105), the cephalad arms (120,120') and adaptable crossbar mounts (175,175') that join the cephalad arms (120,120') to the crossbar (105), interacting with the caudal arms (150,150') which form cups to receive the crossbar (105), is configurable to replace the facets of the spine,
wherein the first cephalad arm (120) comprises a spine reconstruction device (300) for replacing bone, comprising an elongated tubular member (310) with threads (314) on a portion of an exterior of the elongated tubular member (310), and an aperture (319) adapted to communicate with a bone surface.

2. Spinal facet joint prosthesis (100) as claimed in Claim 1, wherein the crossbar (105) has a first end (110) and a second end (115) and the crossbar (105) is a two piece bar where the first end (110) is attached to a threaded male portion having threads and the second end (115) is attached to a threaded female portion sized to receive the threads, the threaded ends allowing for the width of the crossbar (105) to be adjusted to mate with the width between caudal arms (150,150').

3. Spinal facet joint prosthesis (100) as claimed in Claim 1 or Claim 2, wherein each cephalad arm (120,120') includes a bone engaging end (125,125') and a cephalad end (140) adapted to couple to the adaptable crossbar mounts (175, 175').

4. Spinal facet joint prosthesis (100) as claimed in Claim 3, wherein the cephalad end (140) is adapted to engage the crossbar (105) and includes an arm (145) and an elbow (147).

5. Spinal facet joint prosthesis (100) as claimed in Claim 4, wherein the cephalad end (140) is attached to the crossbar (105) using the crossbar mount (175) and the bone engaging end (125) of the second cephalad arm (120') includes a cephalad stem (130) and a distal tip (135), the cephalad stem (130) and the distal tip (135) being configured to engage with each other.

6. Spinal facet joint prosthesis (100) as claimed in Claim 5, wherein the distal tip (135) is formed integrally with the cephalad stem (130).

7. Spinal facet joint prosthesis (100) as claimed in Claim 5 or Claim 6, wherein surface features (132) are provided on the cephalad stem (130) and/or the distal tip (135).

8. Spinal facet joint prosthesis (100) as claimed in Claim 7, wherein the surface features (132,137) are textured surface features to assist in bony in-growth.

9. Spinal facet joint prosthesis (100) as claimed in any one of Claims 1 to 8, wherein each adaptable crossbar mount (175,175') is a connection structure to couple the cephalad arms (120,120') to the crossbar (105).

10. Spinal facet joint prosthesis (100) as claimed in Claim 9, wherein each adaptable crossbar mount (175,175') comprises a cephalad arm engaging portion (172), a crossbar engaging portion (174) and a fixation element (176).

11. Spinal facet joint prosthesis (100) as claimed in Claim 10, wherein fixation element (176) is selected from a screw, stem, cork-screw, wire, staple, or adhesive.

## Patentansprüche

1. Facettengelenk-Prothese (100) der Wirbelsäule, Folgendes umfassend:
einen Querstab (105),
einen ersten und einen zweiten kopfwärtigen Arm (120, 120'), zum Implantieren in Verbindung mit einem ersten Wirbelkörper (14) konfiguriert, und
ein Paar kaudaler Arme (150, 150'), zum Implantieren in Verbindung mit einem zweiten Wirbelkörper (14') konfiguriert;
wobei der zusammenwirkende Einsatz des Querstabs (105), der kopfwärtigen Arme (120, 120') und der anpassbaren Querstabträger (175,175'), die die kopfwärtigen Arme (120, 120') mit dem Querstab (105) verbinden, welcher mit den kaudalen Armen (150, 150'), die Aufnahmebuchsen zum Aufnehmen des Querstabs (105) ausbilden, zusammenwirkt, konfigurierbar ist, um die Facetten der Wirbelsäule zu ersetzen,
wobei der erste kopfwärtige Arm (120) eine Wirbelsäulenrekonstruktionsvorrichtung (300) zum Ersetzen von Knochen umfasst, umfassend ein längliches röhrenförmiges Element (310) mit Gewinde (314) auf einem Abschnitt einer Außenseite des länglichen röhrenförmigen Elements (310) und einer Öffnung (319), die angepasst ist, mit einer Knochenoberfläche zu kommunizieren.

2. Facettengelenk-Prothese (100) der Wirbelsäule nach Anspruch 1, wobei der Querstab (105) ein erstes Ende (110) und ein zweites Ende (115) aufweist und der Querstab (105) ein zweiteiliger Stab ist, wobei das erste Ende (110) an einem Außengewindeteil mit Gewinde befestigt ist und das zweite Ende (115) an einem Innengewindeteil, der bemessen ist, die Gewinde aufzunehmen, befestigt ist, wobei die Gewindeenden es ermöglichen, die Breite des Querstabs (105) anzupassen, um mit der Breite zwischen den kaudalen Armen (150, 150') in Eingriff zu treten.

3. Facettengelenk-Prothese (100) der Wirbelsäule nach Anspruch 1 oder 2, wobei jeder kopfwärtige Arm (120, 120') ein in Knochen eingreifendes Ende (125, 125') und ein kopfwärtiges Ende (140), das angepasst ist, mit den anpassbaren Querstabträgern (175, 175') gekoppelt zu werden, enthält.

4. Facettengelenk-Prothese (100) der Wirbelsäule nach Anspruch 3, wobei das kopfwärtige Ende (140) angepasst ist, in den Querstab (105) einzugreifen, und einen Arm (145) und ein Winkelstück (147) enthält.

5. Facettengelenk-Prothese (100) der Wirbelsäule nach Anspruch 4, wobei das kopfwärtige Ende (140) unter Verwendung des Querstabträgers (175) an dem Querstab (105) befestigt ist und das in Knochen eingreifende Ende (125) des zweiten kopfwärtigen Trägers (120') einen kopfwärtigen Schaft (130) und eine distale Spitze (135) enthält, wobei der kopfwärtige Schaft (130) und die distale Spitze (135) konfiguriert sind, um miteinander in Eingriff zu treten.

6. Facettengelenk-Prothese (100) der Wirbelsäule nach Anspruch 5, wobei die distale Spitze (135) einstückig mit dem kopfwärtigen Schaft (130) ausgebildet ist.

7. Facettengelenk-Prothese (100) der Wirbelsäule nach Anspruch 5 oder 6, wobei Oberflächenmerkmale (132) auf dem kopfwärtigen Schaft (130) und/oder der distalen Spitze (135) bereitgestellt sind.

8. Facettengelenk-Prothese (100) der Wirbelsäule nach Anspruch 7, wobei die Oberflächenmerkmale (132, 137) strukturierte Oberflächenmerkmale sind, um das Einwachsen von Knochen zu unterstützen.

9. Facettengelenk-Prothese (100) der Wirbelsäule nach einem der Ansprüche 1 bis 8, wobei jeder anpassbare Querstabträger (175, 175') eine Verbindungsanordnung ist, um die kopfwärtigen Arme (120, 120') mit dem Querstab (105) zu koppeln.

10. Facettengelenk-Prothese (100) der Wirbelsäule nach Anspruch 9, wobei jeder anpassbare Querstabträger (175, 175') einen in einen kopfwärtigen Arm eingreifenden Abschnitt (172), einen in einen Querstab eingreifenden Abschnitt (174) und ein Befestigungselement (176) umfasst.

11. Facettengelenk-Prothese (100) der Wirbelsäule nach Anspruch 10, wobei ein Befestigungselement (176) aus einer Schraube, einem Schaft, einem Korkenzieher, einem Draht, einem Bügel oder einem Haftmittel ausgewählt ist.

## Revendications

1. Prothèse d'articulation facettaire vertébrale (100) comprenant :
une barre transversale (105),
un premier et un second bras en direction céphalique (120, 120') configurés pour une implantation en conjonction avec un premier corps vertébral (14), et
une paire de bras caudaux (150, 150') configurée pour une implantation en conjonction avec un second corps vertébral (14') ;
dans laquelle le fonctionnement coopératif de la barre transversale (105), des bras en direction céphalique (120, 120') et des supports de barre transversale adaptables (175, 175') qui relient les bras en direction céphalique (120, 120') à la barre transversale (105), interagissant avec les bras caudaux (150, 150') qui forment des coupelles pour recevoir la barre transversale (105), est configurable pour remplacer les facettes de la colonne vertébrale,
dans laquelle le premier bras en direction céphalique (120) comprend un dispositif de reconstruction vertébrale (300) permettant de remplacer de l'os, comprenant un élément tubulaire allongé (310) avec des filets (314) sur une partie d'un extérieur de l'élément tubulaire allongé (310), et une ouverture (319) adaptée pour communiquer avec une surface osseuse.

2. Prothèse d'articulation facettaire vertébrale (100) selon la revendication 1, dans laquelle la barre transversale (105) a une première extrémité (110) et une seconde extrémité (115) et la barre transversale (105) est une barre en deux morceaux où la première extrémité (110) est fixée à une partie mâle filetée ayant des filets et la seconde extrémité (115) est fixée à une partie femelle filetée dimensionnée pour recevoir les filets, les extrémités filetées permettant de régler la largeur de la barre transversale (105) pour qu'elle s'apparie avec la largeur entre les bras caudaux (150, 150').

3. Prothèse d'articulation facettaire vertébrale (100) selon la revendication 1 ou la revendication 2, dans laquelle chaque bras en direction céphalique (120, 120') comporte une extrémité d'engagement d'os (125, 125') et une extrémité en direction céphalique (140) adaptée pour s'accoupler aux supports de barre transversale adaptables (175, 175').

4. Prothèse d'articulation facettaire vertébrale (100) selon la revendication 3, dans laquelle l'extrémité en direction céphalique (140) est adaptée pour engager la barre transversale (105) et comporte un bras (145) et un coude (147).

5. Prothèse d'articulation facettaire vertébrale (100) selon la revendication 4, dans laquelle l'extrémité en direction céphalique (140) est fixée à la barre transversale (105) à l'aide du support de barre transversale (175) et l'extrémité d'engagement d'os (125) du second bras en direction céphalique (120') comporte une tige en direction céphalique (130) et une pointe distale (135), la tige en direction céphalique (130) et la pointe distale (135) étant configurées pour s'engager l'une l'autre.

6. Prothèse d'articulation facettaire vertébrale (100) selon la revendication 5, dans laquelle la pointe distale (135) est formée d'un seul tenant avec la tige en direction céphalique (130).

7. Prothèse d'articulation facettaire vertébrale (100) selon la revendication 5 ou la revendication 6, dans laquelle les particularités de surface (132) sont prévues sur la tige en direction céphalique (130) et/ou la pointe distale (135).

8. Prothèse d'articulation facettaire vertébrale (100) selon la revendication 7, dans laquelle les particularités de surface (132, 137) sont des particularités de surface texturées pour aider la croissance osseuse.

9. Prothèse d'articulation facettaire vertébrale (100) selon l'une quelconque des revendications 1 à 8, dans laquelle chaque support de barre transversale adaptable (175, 175') est une structure de raccordement pour accoupler les bras en direction céphalique (120, 120') à la barre transversale (105).

10. Prothèse d'articulation facettaire vertébrale (100) selon la revendication 9, dans laquelle chaque support de barre transversale adaptable (175, 175') comprend une partie d'enclenchement de bras en direction céphalique (172), une partie d'enclenchement de barre transversale (174) et un élément de fixation (176).

11. Prothèse d'articulation facettaire vertébrale (100) selon la revendication 10, dans laquelle l'élément de fixation (176) est choisi parmi une vis, une tige, une vrille, du fil, une agrafe ou un adhésif.
